# FASCICULE DE BREVET EUROPEEN

(11) **EP 1 486 503 B1**
(45) Date de publication et mention de la délivrance du brevet: **02.11.2006**
(21) Numéro de dépôt: 04291435.8
(22) Date de dépôt: 09.06.2004
(51) Int. Cl.: C07D 513/04, A61K 31/54, A61P 25/28

(54) **Dérivés de benzothiazine et benzothiadiazine, leur procédé de préparation et les compositions pharmaceutiques qui les contiennent**
Derivate von Benzothiazin und Benzothiadiazin, Verfahren zu ihrer Herstellung und pharmazeutische Zubereitungen diese enthaltend
Derivatives of benzothiazine and benzothiadiazine, procedure for their preparation and pharmaceutical compositions containing them

(30) Priorité: 13.06.2003 FR 0307118
(43) Date de publication de la demande: 15.12.2004
(73) Titulaire: Les Laboratoires Servier, 92415 Courbevoie Cedex (FR)
(72) Inventeur: Desos, Patrice, 92270 Bois-Colombes (FR); Cordi, Alex, 92150 Suresnes (FR); Lestage, Pierre, 78170 La Celle Saint Cloud (FR)

(56) Documents cités:
- EP-A- 0 692 484
- WO-A-99/42456
- WO-A-03/053947
- FR-A- 1 377 693

## Description

La présente invention concerne de nouveaux dérivés de benzothiazine et benzothiadiazine, leur procédé de préparation et les compositions pharmaceutiques qui les contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit entre autres certains dérivés de benzothiadiazine en tant que modulateurs des récepteurs AMPA.

Les dérivés de benzothiazine et de benzothiadiazine, objets de la présente invention, outre le fait qu'ils soient nouveaux, présentent, de manière surprenante, des activités pharmacologiques sur le courant AMPA nettement supérieures à celles des composés de structures proches décrits dans l'Art Antérieur. Ils sont utiles en tant que modulateurs AMPA pour le traitement ou la prévention des désordres mnémocognitifs associés à l'âge, aux syndromes anxieux ou dépressifs, aux maladies neurodégénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule **(I)** : 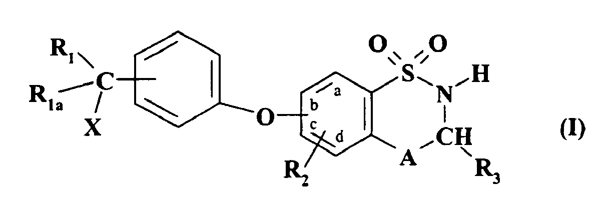 dans laquelle :
- **R**_{**1**}: représente un atome d'hydrogène, d'halogène ou un groupement alkyle (C₁-C₆) linéaire ou ramifié,
- **R**_{**1a**}: représente un atome d'hydrogène ou un groupement alkyle (C₁-C₆) linéaire ou ramifié,
- **R**_{**2**}: représente un atome d'hydrogène, d'halogène ou un groupement hydroxy,
- **A**: représente un groupement CR₄R₅ ou un groupement NR₄,
- **R**_{**3**}: représente un atome d'hydrogène, un groupement alkyle (C₁-C₆) linéaire ou ramifié ou un groupement cycloalkyle (C₃-C₇),
- **R**_{**4**}: représente un atome d'hydrogène ou un groupement alkyle (C₁-C₆) linéaire ou ramifié,
ou bien
- **A**: représente un atome d'azote et forme avec le groupement -CHR₃- adjacent le cycle 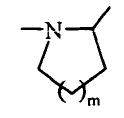 dans lequel m représente 1, 2 ou 3,
- **R**_{**5**}: représente un atome d'hydrogène ou d'halogène,
- **X**: représente un groupement NR₆R₇, S(O)ₙR₈, OR'₈ ou un groupement hétérocyclique dans lesquels :
**R**_{**6**} représente un atome d'hydrogène, un groupement alkyle (C₁-C₆) linéaire ou ramifié, S(O)ₚR₉, COR₉ ou P(O)OR₉OR₁₀,
**R**_{**7**} représente un atome d'hydrogène, ou un groupement alkyle (C₁-C₆) linéaire ou ramifié,
ou bien **R**_{**6**} et **R**_{**7**} forment ensemble avec l'atome d'azote qui les porte un groupement hétérocyclique,
**R**_{**8**}**, R**_{**9**} et **R**_{**10**}**,** identiques ou différents, représentent un atome d'hydrogène ; un groupement alkyle (C₁-C₆) linéaire ou ramifié éventuellement substitué par un ou plusieurs atomes d'halogène ; un groupement arylalkyle (C₁-C₆) linéaire ou ramifié ; ou un groupement aryle,
**R'**_{**8**} représente un groupement alkyle (C₁-C₆) linéaire ou ramifié ou acyle (C₁-C₆) linéaire ou ramifié,
n et p, identiques ou différents, représentent 1 ou 2.
leurs énantiomères et diastéréoisomères ainsi que leurs sels d'addition à un acide ou à une base pharmaceutiquement acceptable,
étant entendu que :
◆ par groupement hétérocyclique, on comprend groupement aromatique ou non, monocyclique ou bicyclique, contenant un à quatre hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, alkoxycarbonyle (C₁-C₆) linéaire ou ramifié, oxo, thioxo, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), et alkyl (C₁-C₆) sulfonylamino,
◆ par groupement aryle, on comprend groupement aromatique monocyclique ou un groupement bicyclique dans lequel au moins un des cycles est aromatique, éventuellement substitué par un ou plusieurs groupements, identiques ou différents, choisis parmi halogène, alkyle (C₁-C₆) linéaire ou ramifié (éventuellement substitué par un ou plusieurs groupements hydroxy), alkoxy (C₁-C₆) linéaire ou ramifié, polyhalogénoalkyle (C₁-C₆) linéaire ou ramifié, alkoxy carbonyle (C₁-C₆) linéaire ou ramifié, oxo, thioxo, alkylthio (C₁-C₆) linéaire ou ramifié, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié ou acyle (C₁-C₆) linéaire ou ramifié), aminocarbonyle (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), mono ou di(alkyl(C₁-C₆)sulfonyl)amino, mono ou di(trifluorométhylsulfonyl)amino, PO(ORₐ)(OR_{b}) (dans lequel Rₐ, R_{b}, identiques ou différents, représentent un atome d'hydrogène ou un groupement alkyle (C₁-C₆) linéaire ou ramifié), benzyloxy, et phényle (éventuellement substitué par un ou plusieurs groupements, identiques ou différents, choisis parmi halogène, alkyle (C₁-C₆) linéaire ou ramifié, perhalogénoalkyle (C₁-C₆) linéaire ou ramifié, hydroxy et alkoxy (C₁-C₆) linéaire ou ramifié).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Le groupement 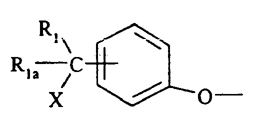 est préférentiellement en position b du phényle qui le porte.

Les groupements R₁ₐ et R₂ préférés sont des atomes d'hydrogène.

Le groupement 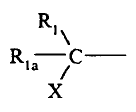 est préférentiellement en position méta ou para du noyau phénoxy qui le porte.

X représente préférentiellement un groupement NR₆R₇, S(O)ₙR₈ ou un groupement hétérocyclique.

Plus particulièrement, le groupement X préféré est le groupement NR₆R₇ dans lequel R₆ représente un atome d'hydrogène ou un groupement S(O)ₚR₉ et R₇ représente un atome d'hydrogène, comme par exemple les groupements NHSO₂Me, NHSO₂iPr, NHSO₂CF₃, NH₂.

Les composés préférés de l'invention sont les composés tels que A représente un atome d'azote et forme avec le groupement 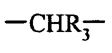 adjacent le cycle 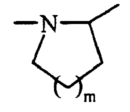 dans lequel m représente 1, 2 ou 3 et préférentiellement 1.

Les composés préférés de l'invention sont :
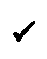 le {3-[(5,5-dioxido-2,3,3a,4-tétrahydro-1*H*-pyrrolo[2,1-*c*][1,2,4]benzothiadiazin-7-yl) oxy]phényl}méthanamine,
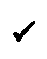 le *N-*{3-[(5,5-dioxido-2,3,3a,4-tétrahydro-1*H*-pyrrolo[2,1-*c*][1,2,4]benzothiadiazin-7-yl) oxy]benzyl}méthanesulfonamide,
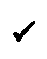 le *N-*{4-[(5,5-dioxido-2,3,3a,4-tétrahydro-1*H* pyrrolo[2,1-*c*][1,2,4]benzothiadiazin-7-yl) oxy]benzyl}méthanesulfonamide.

L'invention s'étend également au procédé de préparation des composés de formule (I).

Le procédé de préparation des composés de formule (I) dans laquelle A représente un groupement NR₄ ou A représente un atome d'azote et forme avec le groupement CHR₃ adjacent le cycle 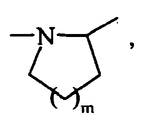 dans lequel m représente 1, 2 ou 3, est caractérisé en ce que l'on utilise comme produit de départ un composé de formule **(II)** : 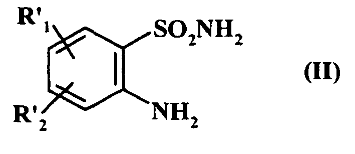 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui est :
**(a) soit mis en réaction** avec le chlorure d'acide de formule **(III)** en présence d'une base, en milieu tétrahydrofurane ou acétonitrile :

   **Cl - CH**_{**2**} **- (CH**_{**2**}**)**_{**m**} **- CH**_{**2**} **- COCl** **(III)**

   dans laquelle m a la même signification que dans la formule (I),
   pour conduire au composé de formule **(IV) :** 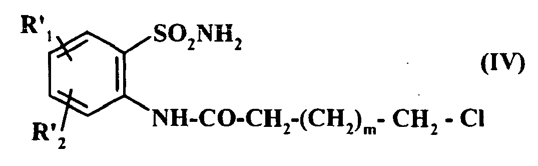 dans laquelle R'₁ et R'₂ sont tels que définis précédemment,
   qui est alors cyclisé en milieu basique, pour conduire au composé de formule **(V) :** 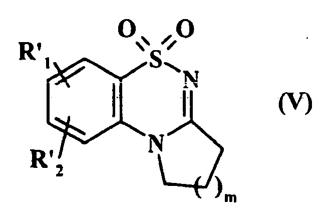 dans laquelle R'₁, R'₂ et m sont tels que définis précédemment,
   qui subit éventuellement une réduction, en milieu alcoolique ou diméthylformamide, en présence de borohydrure de sodium, pour conduire au composé de formule **(VI)** : 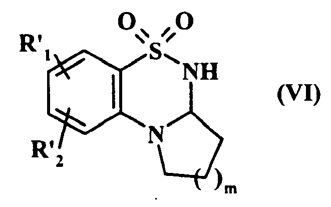 dans laquelle R'₁, R'₂ et m sont tels que définis précédemment,
   composé de formule **(V)** ou **(VI)** qui subit l'action du tribromure de bore,
   pour conduire au composé de formule **(VII)** : 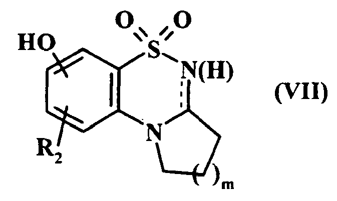 dans laquelle R₂ est tel que défini dans la formule (I) et m est tel que défini précédemment,
**(b) soit cyclisé :**
   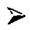 en présence d'une amidine de formule **(VIII) :** 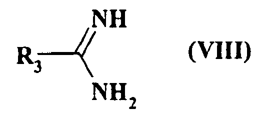 dans laquelle R₃ est tel que défini dans la formule (I), pour conduire au composé de formule **(IX) :** 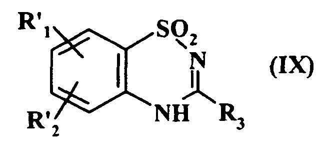 dans laquelle R'₁, R'₂, et R₃ ont la même signification que précédemment,
      qui est :
      ◆ ou bien réduit par un hydrure métallique,
         pour conduire au composé de formule **(X)** : 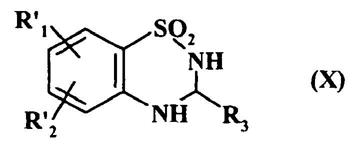 dans laquelle R'₁, R'₂, et R₃ ont la même signification que précédemment,
      ◆ ou bien alkylé par action d'une base forte en présence d'un agent d'alkylation R'₄X dans lequel R'₄ représente un groupement alkyle (C₁-C₆) linéaire ou ramifié et X un atome d'halogène, puis réduit, pour conduire au composé de formule (XI) : 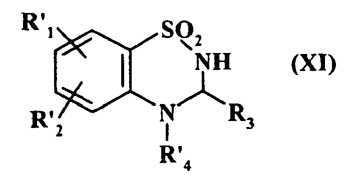 dans laquelle R'₁, R'₂, R₃ et R'₄ ont la même signification que précédemment,
   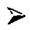 en présence d'un aldéhyde de formule **(XII)** : 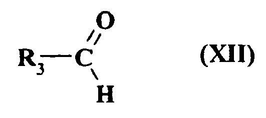 dans laquelle R₃ est tel que défini dans la formule (I),
      pour conduire au composé de formule (X) décrit précédemment,
      composé de formule (X) ou (XI),
      dont on transforme le groupement R'₁ et le groupement R'₂ lorsque celui-ci représente un groupement alkoxy (C₁-C₆) linéaire ou ramifié, en groupements hydroxy,
      pour conduire au composé de formule **(XIII) :** 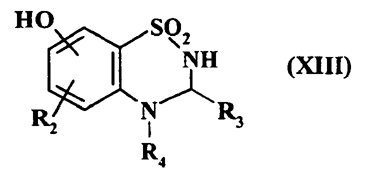 dans laquelle R₂, R₃ et R₄ ont la même signification que dans la formule (I),
      composé de formule (VII) ou (XIII) que l'on fait réagir avec un dérivé d'acide boronique de formule **(XIV)** : 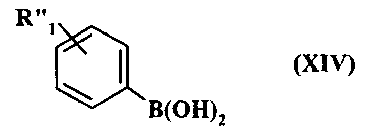 dans laquelle R"₁ représente un groupement cyano ou un groupement R₁R₁ₐXC- tel que défini dans la formule (I),
      pour conduire, (après transformation éventuelle du groupement R"₁ quand celui-ci représente un groupement cyano en groupement NR₆R₇ tel que défini dans la formule (I)), au composé de formule **(I/a**_{**1**}**)** ou **(I/a**_{**2**}**),** cas particuliers des composés de formule (I) : 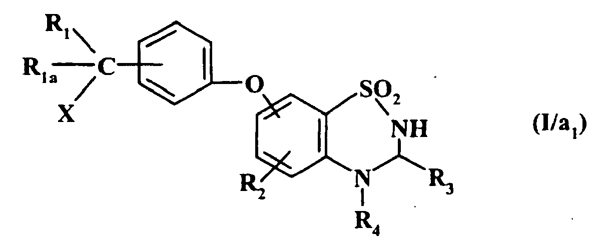 dans laquelle R₁, R₁ₐ, R₂, R₃, R₄ et X ont la même signification que dans la formule (I), 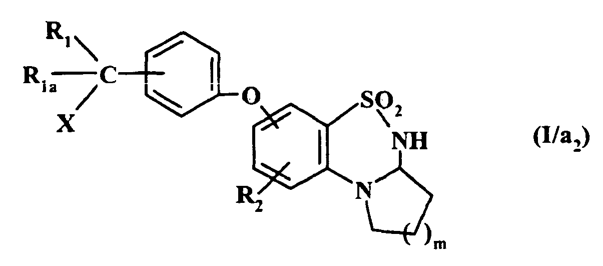 dans laquelle R₁, R₁ₐ, R₂, m et X ont la même signification que dans la formule (I),
      composés de formule (I/a₁) ou (I/a₂) :
      que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

Le procédé de préparation des composés de formule (I) dans laquelle A représente un groupement CR₄R₅ est caractérisé en ce que l'on utilise comme produit de départ, un composé de formule **(XV) :** 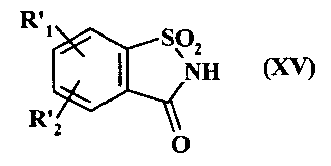 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui subit l'action de la chloroacétone en présence de diméthylformamide, pour conduire au composé de formule **(XVI)** : 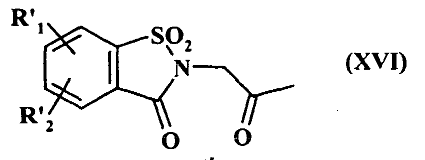 dans laquelle R'₁ et R'₂ ont la même signification que précédemment,
qui subit un réarrangement en milieu basique, pour conduire au composé de formule **(XVII) :** 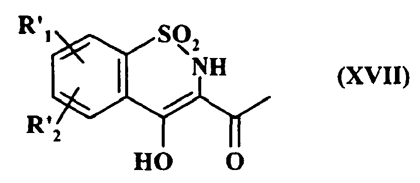 dans laquelle R'₁ et R'₂ ont la même signification que précédemment,
qui est déacétylé par chauffage au reflux en milieu benzénique en présence d'un excès d'éthylèneglycol et d'une quantité catalytique d'acide *p*-toluène sulfonique, pour conduire au composé de formule (**XVIII**) : 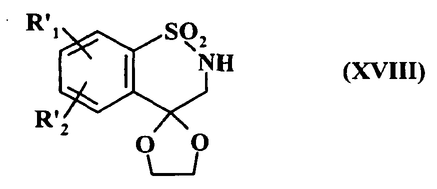 dans laquelle R'₁ et R'₂ ont la même signification que précédemment,
qui subit une hydrolyse en milieu acide, pour conduire au composé de formule **(XIXa) :** 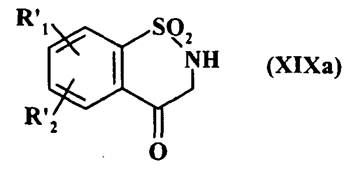 dans laquelle R'₁ et R'₂ ont la même signification que précédemment,
dont, éventuellement, selon la nature du groupement R₃ que l'on souhaite obtenir, on protège l'atome d'azote par un groupement protecteur, puis, après traitement par une base forte, que l'on traite par un composé de formule R'₃-P,
dans laquelle R'₃ représente un groupement alkyle (C₁-C₆) linéaire ou ramifié ou un groupement cycloalkyle (C₃-C₇) et P représente un groupe partant, pour conduire après déprotection de l'atome d'azote, au composé de formule **(XIX'a)** : 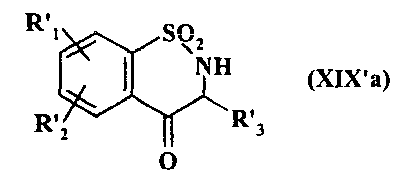 dans laquelle R'₁, R'₂ et R'₃ ont la même signification que précédemment,
composé de formule (XIXa) et (XIX'a) représentés par la formule **(XIX) :** 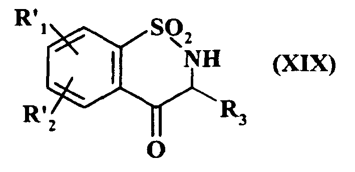 dans laquelle R'₁ et R'₂ ont la même signification que précédemment et R₃ est tel que défini dans la formule (I), qui :
- **soit** subit une réduction catalytique, pour conduire au composé de formule **(XX)** : 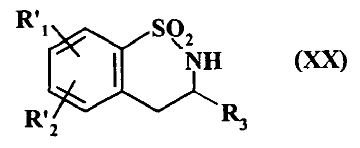 dans laquelle R'₁ et R'₂ ont la même signification que précédemment,
- **soit** est transformé par l'action d'un hydrure en alcool dont on transforme le groupement hydroxy en atome d'halogène par l'action d'un réactif approprié,
   pour conduire au composé de formule **(XXI)** : 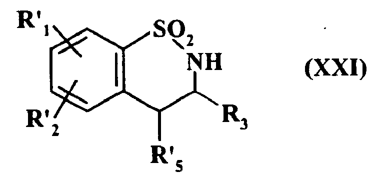 dans laquelle R'₁, R'₂ et R₃ ont la même signification que précédemment, et R'₅ représente un atome d'halogène,
- **soit** subit l'action d'un organomagnésien R'₄MgBr dans lequel R'₄ représente un groupement alkyle (C₁-C₆) linéaire ou ramifié,
   pour conduire au composé de formule **(XIXb)** : 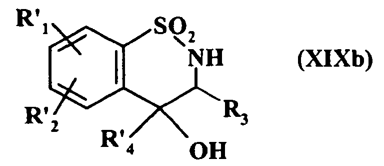 dans laquelle R'₁, R'₂, R₃ et R'₄ ont la même signification que précédemment,
   composé de formule **(XIXb)** :
- **ou bien** qui subit une réduction catalytique, pour conduire au composé de formule (**XXII**) : 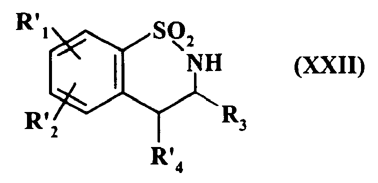 dans laquelle R'₁, R'₂, R₃ et R'₄ ont la même signification que précédemment,
- **ou bien** dont on transforme le groupement hydroxy en atome d'halogène par l'action d'un réactif approprié,
   pour conduire au composé de formule **(XXIII)** : 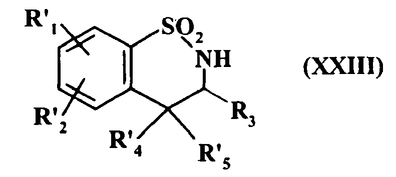 dans laquelle R'₁, R'₂, R₃ et R'₄ ont la même signification que précédemment et R'₅ représente un atome d'halogène,
   composé de formule **(XX)** à **(XXIII)** dont on transforme le groupement R'₁ et le groupement R'₂ lorsque celui-ci représente un groupement alkoxy (C₁-C₆) linéaire ou ramifié, en groupements hydroxy, pour conduire au composé de formule **(XXIV)** : 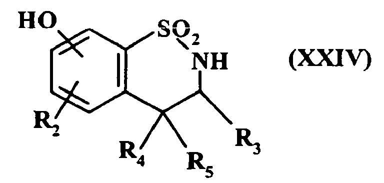 dans laquelle R₂, R₃, R₄ et R₅ ont la même signification que dans la formule (I),
   composé de formule **(XXIV),**
   que l'on fait réagir avec un dérivé d'acide boronique de formule **(XIV)** : 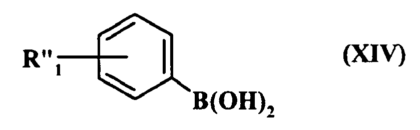 dans laquelle R"₁ représente un groupement cyano ou un groupement R₁R₁ₐXC- tel que défini dans la formule (I),
   pour conduire, (après transformation éventuelle du groupement R"₁ quand celui-ci représente un groupement cyano en groupement NR₆R₇ tel que défini dans la formule (I)), au composé de formule **(I/b),** cas particuliers des composés de formule (I) : 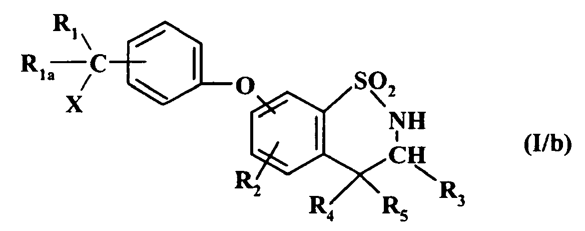 dans laquelle R₁, R₁ₐ, R₂, R₃, R₄, R₅ et X ont la même signification que dans la formule (I),
   composé de formule (I/b), que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse ...).

### EXEMPLE 1 : {3-[(5,5-Dioxido-2,3,3a,4-tétrahydro-1H-pyrrolo[2,1-c][1,2,4] benzothiadiazin-7-yl)oxy]phényl}méthanamine, chlorhydrate

### Stade A : 2,3-Dihydro-1H-pyrrolo[2,1-c][1,2,4]benzothiadiazin-7-ol 5,5-dioxide

A une solution de 27,5 mmoles de 7-methoxy-2,3-dihydro-1*H*-pyrrolo[2,1-*c*] [1,2,4]benzothiadiazine 5,5-dioxide dans 350 ml de chlorure de méthylène refroidi à 0°C, on additionne goutte à goutte une solution de BBr₃ (68,75 mmoles) dans 25 ml de chlorure de méthylène. On poursuit l'agitation 24 h à température ambiante. La réaction est versée dans un mélange glace/eau et la suspension est agitée 30 min Le précipité est filtré, rincé plusieurs fois avec de l'eau, essoré et séché sous vide pour donner le produit attendu.
*Point de fusion : > 300 °C*

**Micro-analyse élémentaire :**

| | *C%* | *H%* | *N%* | *S%* |
|---|---|---|---|---|
| *Calculée* | *50,41* | *4,23* | *11,76* | *13,46* |
| *Trouvée* | *50, 00* | *4,19* | *11, 28* | *13,41* |

### Stade B : 3-[(5,5-Dioxido-2,3-dihydro-1H-pyrrolo[2,1-c][1,2,4]benzothiadiazin-7-yl) oxy]benzonitrile

Dans 200 ml de chlorure de méthylène on agite pendant 24 h une suspension composée de 7,06 mmoles du produit obtenu au stade précédent, d'acide 3-cyanophényl boronique (11,02 mmoles), d'acétate de cuivre (II) (11,02 mmoles), de pyridine (22,0 mmoles) et d'environ 500 mg de tamis moléculaire 4 Å. Le milieu réactionnel est dilué en ajoutant 100 ml supplémentaires de chlorure de méthylène et on filtre la suspension. Le filtrat est concentré puis directement déposé sur une colonne de silice que l'on élue avec un système 95/5 chlorure de méthylène/méthanol. Les fractions contenant le produit attendu sont rassemblés, évaporés et le résidu est repris dans un peu d'éther éthylique. Après filtration du solide on récupère le produit attendu sous forme de poudre blanche.
*Point de fusion : 229-233 °C*

**Micro-analyse élémentaire :**

| | *C%* | *H%* | *N%* | *S%* |
|---|---|---|---|---|
| *Calculée* | *60,17* | *3,86* | *12,38* | *9,45* |
| *Trouvée* | *59,42* | *3,96* | *12,29* | *9,63* |

### Stade C : {3-[(5,5-Dioxido-2,3,3a,4-tétrahydro-1H-pyrrolo[2,1-c][1,2,4] benzothiadiazin-7-yl)oxy]phényl}méthanamine, chlorhydrate

A une solution du produit du stade précédent 0,58 mmoles dans 20 ml de THF anhydre, on rajoute par petites portions 112 mg (2,95 mmoles) de LiAlH₄ et on agite le mélange 1 h à température ambiante. L'excès d'hydrure est hydrolysé par addition successive et goutte à goutte de 1.5 ml d'isopropanol et 1.5 ml d'une solution aqueuse saturée en NaCl. On filtre les sels d'alumines et le filtrat est évaporé à sec. Le résidu est chromatographié sur colonne de silice en éluant avec un mélange CH₂Cl₂/EtOH/NH₃ aq 95/5/0.5. Après évaporation des fractions contenant l'amine, la meringue est reprise dans de l'éther chlorhydrique. La solution est évaporée à sec et le résidu est redissous dans un minimum d'isopropanol. Le produit attendu cristallise et est récupéré par filtration.
*Point de fusion : 145 °C*

**Micro-analyse élémentaire :**

| | *C%* | *H%* | *N%* | *S%* | *Cl%* |
|---|---|---|---|---|---|
| *Calculée* | *53,47* | *5,28* | *11,00* | *8,40* | *9,28* |
| *Trouvée* | *53, 09* | *5,34* | *10, 65* | *8,30* | *9,30* |

### EXEMPLE 2 : N-{3-[(5,5-Dioxido-2,3,3a,4-tétrahydro-1H-pyrrolo[2,1-c][1,2,4] benzothiadiazin-7-yl)oxy]benzyl}méthanesulfonamide

A une solution du composé obtenu dans l'exemple 1 (0,136 mmoles) dans 10 ml de CH₂Cl₂ refroidie dans un bain de glace contenant 0,34 mmoles de Et₃N on ajoute, goutte à goutte, l'anhydride méthane sulfonique (0,20 mmoles) en solution dans 2 ml de CH₂Cl₂. La réaction est agitée 3 h à température ambiante. La solution réactionnelle est lavée à l'eau puis par NaCl saturée et est séchée sur MgSO₄. Après évaporation sous vide, le résidu est concrétisé par trituration dans l'éther éthylique pour conduire après filtration au produit du titre.
*Point de fusion : 183-190 °C*

**Micro-analyse élémentaire :**

| | *C%* | *H%* | *N%* | *S%* |
|---|---|---|---|---|
| *Calculée* | *51,06* | *5,00* | *9,92* | *15,14* |
| *Trouvée* | *51,09* | *5,33* | *9,58* | *15,55* |

Les exemples suivants ont été préparés selon les procédés décrits dans les exemples 1 ou 2 à partir des produits de départ correspondants.

### EXEMPLE 3 : N-{3-[(5,5-Dioxido-2,3,3a,4-tétrahydro-1H-pyrrolo[2,1-c][1,2,4] benzothiadiazin-7-yl)oxy]benzyl}acétamide

*Point de fusion : 58 °C*

**Micro-analyse élémentaire :**

| | *C%* | *H%* | *N%* | *S%* |
|---|---|---|---|---|
| *Calculée* | *58,90* | *5,46* | *10,85* | *8,28* |
| *Trouvée* | *58.51* | *5.73* | *10,36* | *7,82* |

### EXEMPLE 4 : N-(1-{3-[(5,5-Dioxido-2,3,3a,4-tétrahydro-1H-pyrrolo[2,1-c][1,2,4] benzothiadiazin-7-yl)oxy]phényl}éthyl)acétamide

### EXEMPLE 5 : N-(1-{3-[(5,5-Dioxido-2,3,3a,4-tétrahydro-1H-pyrrolo[2,1-c][1,2,4] benzothiadiazin-7-yl)oxy]phényl}-1-fluoroéthyl)acétamide

### EXEMPLE 6 : 3-[(5,5-Dioxido-2,3,3a,4-tétrahydro-1H-pyrrolo[2,1-c][1,2,4] benzothiadiazin-7-yl)oxy]benzyl isopropyl sulfone

### EXEMPLE 7 : 1-{3-[(5,5-Dioxido-2,3,3a,4-tétrahydro-1H-pyrrolo[2,1-c][1,2,4] benzothiadiazin-7-yl)oxy]phényl}éthyl isopropyl sulfone

### EXEMPLE 8 : 1-{3-[(5,5-Dioxido-2,3,3a,4-tétrahydro-1H-pyrrolo[2,1-c][1,2,4] benzothiadiazin-7-yl)oxy]phényl}-1-fluoroéthyl isopropyl sulfone

### EXEMPLE 9 : N-{3-[(5,5-Dioxido-2,3,3a,4-tétrahydro-1H-pyrrolo[2,1-c][1,2,4] benzothiadiazin-7-yl)oxy]benzyl}(trifluoro)méthanesulfonamide

*Point de fusion : 104-113 °C*

**Micro-analyse élémentaire :**

| | *C%* | *H%* | *N%* | *S%* |
|---|---|---|---|---|
| *Calculée* | *45,28* | *3,80* | *8,80* | *13,43* |
| *Trouvée* | *46,20* | *3,87* | *8,58* | *13,84* |

### EXEMPLE 10 : N-(1-{3-[(5,5-Dioxido-2,3,3a,4-tétrahydro-1H-pyrrolo[2,1-c][1,2,4] benzothiadiazin-7-yl)oxy]phényl}éthyl)(trifluoro) méthanesulfonamide

### EXEMPLE 11 : N-(1-{3-[(5,5-Dioxido-2,3,3a,4-tétrahydro-1H-pyrrolo[2,1-c][1,2,4] benzothiadiazin-7-yl)oxy]phényl}-1-fluoroéthyl)(trifluoro) méthanesulfonamide

### EXEMPLE 12 : N-{3-[(5,5-Dioxido-2,3,3a,4-tétrahydro-1H-pyrrolo[2,1-c][1,2,4] benzothiadiazin-7-yl)oxy]benzyl}-N-méthylbenzamide

### EXEMPLE 13 : N-(1-{3-[(5,5-Dioxido-2,3,3a,4-tétrahydro-1H-pyrrolo[2,1-c][1,2,4] benzothiadiazin-7-yl)oxy]phényl}éthyl)-N-méthylbenzamide

### EXEMPLE 14 : N-(1-{3-[(5,5-Dioxido-2,3,3a,4-tétrahydro-1H-pyrrolo[2,1-c][1,2,4] benzothiadiazin-7-yl)oxy]phényl}-1-fluoroéthyl)-N-méthylbenzamide

### EXEMPLE 15 : 7-{[3-(1H-Imidazol-4-yl)méthyl]phénoxy}-2,3,3a,4-tétrahydro-1H-pyrrolo[2,1-c][1,2,4]benzothiadiazin-5,5-dioxide

### EXEMPLE 16 : 7-{3-[1-(1H-Imidazol-4-yl)éthyl]phénoxy}-2,3,3a,4-tétrahydro-1H-pyrrolo[2,1-c][1,2,4]benzothiadiazin-5,5-dioxide

### EXEMPLE 17 : 7-{3-[1-Fluoro-1-(1H-imidazol-4-yl)éthyl]phénoxy}-2,3,3a,4-tétrahydro-1H-pyrrolo[2,1-c][1,2,4]benzothiadiazin-5,5-dioxide

### EXEMPLE 18 : N-{3-[(5,5-Dioxido-2,3,3a,4-tétrahydro-1H-pyrrolo[2,1-c][1,2,4] benzothiadiazin-7-yl)oxy]benzyl}propane-2-sulfonamide

*Point de fusion : 112-118 °C*

**Micro-analyse élémentaire :**

| | C | *H* | *N* | *S* |
|---|---|---|---|---|
| *% théorique* | *53,20* | *5,58* | *9,31* | *14,20* |
| *% expérimental* | *53,36* | *5,80* | *9,24* | *14,52* |

### EXEMPLE 19 : 7-[3-(1H-Pyrrol-1-ylméthyl)phénoxy]-2,3,3a,4-tétrahydro-1H-pyrrolo [2,1-c][1,2,4]benzothiadiazine 5,5-dioxide

Dans un mélange biphasique de 2,5 ml d'eau, 0,95 ml d'AcOH, 2,85 ml de dichloro1,2-éthane, on ajoute 200 mg (0,58 mmol) du produit obtenu dans l'exemple 1 sous forme d'amine libre et 105 µl (0,81 mmol) de 2,5 diméthoxytétrahydrofurane. On agite 2 h à 80°C, laisse revenir à température ambiante et extrait avec CH₂Cl₂. La phase organique est lavée par une solution aqueuse saturée de NaCl, séchée sur MgSO₄. Le produit attendu est purifié par chromatographie sur colonne de silice (CH₂Cl₂/heptane 75/25).
*Point de fusion : 150-152 °C*

**Micro-analyse élémentaire :**

| | *C* | *H* | *N* | *S* |
|---|---|---|---|---|
| *% théorique* | *63,78* | *5,35* | *10,63* | *8,11* |
| *% expérimental* | *63,65* | *5,28* | *10,38* | *8,41* |

### EXEMPLE 20 : 1-{4-[(5,5-Dioxido-2,3,3a,4-tétrahydro-1H-pyrrolo[2,1-c][1,2,4] benzothiadiazin-7-yl)oxy]phényl}méthanamine, chlorhydrate

On procède comme dans l'exemple 1 en remplaçant au stade B l'acide 3-cyanophényl boronique par l'isomère 4-cyanophényl boronique.
*Point de fusion : 165-172 °C*

**Micro-analyse élémentaire :**

| | *C* | *H* | *N* | *S* | *Cl* |
|---|---|---|---|---|---|
| *% théorique* | *53,47* | *5,28* | *11,00* | *8,40* | *9,28* |
| *% expérimental* | *54,08* | *5,16* | *10,46* | *8,25* | *9,42* |

### EXEMPLE 21 : N-{4-[(5,5-Dioxido-2,3,3a,4-tétrahydro-1H-pyrrolo[2,1-c][1,2,4] benzothiadiazin-7-yl)oxy]benzyl}méthanesulfonamide

On procède comme dans l'exemple 2 à partir du composé obtenu dans l'exemple 20.
*Point de fusion : 104-110 °C*

**Micro-analyse élémentaire :**

| | *C* | *H* | *N* | *S* |
|---|---|---|---|---|
| *% théorique* | *51,05* | *5,00* | *9,92* | *15,14* |
| *% expérimental* | *51,24* | *5,45* | *9,17* | *14,94* |

Les 2 énantiomères de l'exemple 21 ont été séparés par chomatographie chirale sur colonne Chiralpak AD^{®}. Elution : CH₃CN/iPrOH/DEA 1000/2/1. Les 2 énantiomères sont exemplifiés dans les exemples 22 et 23 par ordre d'élution selon les conditions mentionnées ci-dessus.

### EXEMPLE 22 : N-{4-[(5,5-Dioxido-2,3,3a,4-tétrabydro-1H-pyrrolo[2,1-c][1,2,4] benzothiadiazin-7-yl)oxy]benzyl}méthanesulfonamide, énantiomère 1

**Micro-analyse élémentaire :**

| | *C* | *H* | *N* | *S* |
|---|---|---|---|---|
| *% théorique* | *51,05* | *5,00* | *9,92* | *15,14* |
| *% expérimental* | *50,48* | *5,08* | *9, 63* | *15,53* |

### EXEMPLE 23 : N-{4-[(5,5-Dioxido-2,3,3a,4-tétrahydro-1H-pyrrolo[2,1-c][1,2,4] benzothiadiazin-7-yl)oxy]benzyl}méthanesulfonamide, énantiomère 2

**Micro-analyse élémentaire:**

| | *C* | *H* | *N* | *S* |
|---|---|---|---|---|
| *% théorique* | *51,05* | *5,00* | *9,92* | *15,14* |
| *% expérimental* | *50,77* | *5, 06* | *9,70* | *15,47* |

### EXEMPLE 24 : N-{4-[(5,5-Dioxido-2,3,3a,4-tétrahydro-1H-pyrrolo[2,1-c][1,2,4] benzothiadiazin-7-yl)oxy]benzyl}acétamide

On procède comme dans l'exemple 2 en remplaçant l'anhydride méthane sulfonique par le chlorure d'acétyle et en utilisant comme produit de départ l'amine obtenue dans l'exemple 20.
*Point de fusion : 158-161 °C*

**Micro-analyse élémentaire :**

| | | | | |
|---|---|---|---|---|
| | *C* | *H* | *N* | *S* |
| *% théorique* | *58,90* | *5,46* | *10,85* | *8,28* |
| *% expérimental* | *58,85* | *5,69* | *10,65* | *8,51* |

### EXEMPLE 25 : N-{4-[(5,5-Dioxido-2,3,3a,4-tétrahydro-1H-pyrrolo[2,1-c][1,2,4] benzothiadiazin-7-yl)oxy]benzyl}-2,2,2-trifluoroacétamide

On procède comme dans l'exemple 2 en remplaçant l'anhydride méthane sulfonique par l'anhydride trifluoro acétique et en utilisant comme produit de départ l'amine obtenue dans l'exemple 20.
*Point de fusion : 136-138 °C*

**Micro-analyse élémentaire :**

| | *C* | *H* | *N* | *S* |
|---|---|---|---|---|
| *% théorique* | *51,70* | *4,11* | *9,52* | *7,26* |
| *% expérimental* | *51,84* | *4,24* | *9,36* | *7,48* |

### EXEMPLE 26 : N-{3-[(5,5-Dioxido-2,3,3a,4-tétrahydro-1H-pyrrolo[2,1-c][1,2,4] benzothiadiazin-7-yl)oxy]benzyl}-4-fluorobenzamide

On procède comme dans l'exemple 2 en remplaçant l'anhydride méthane sulfonique par le chlorure de 4-fluorobenzoyle et en utilisant comme produit de départ l'amine obtenue dans l'exemple 20.
*Point de fusion : 104-108 °C*

**Micro-analyse élémentaire :**

| | *C* | *H* | *N* | *S* |
|---|---|---|---|---|
| *% théorique* | *61,66* | *4,74* | *8,99* | *6,86* |
| *% expérimental* | *61,41* | *4,81* | *8,72* | *6,66* |

### EXEMPLE 27 : 7-[4-(1H-Tétrazol-5-ylméthyl)phénoxyl-2,3,3a,4-tétrahydro-1H-pyrrolo[2,1-c][1,2,4]benzothiadiazine 5,5-dioxide

### Stade A : {4-[(5,5-Dioxido-2,3,3a,4-tétrahydro-1H-pyrrolo[2,1-c][1,2,4] benzothiadiazin-7-yl)oxy]phényl}acétonitrile

Dans 200 ml de chlorure de méthylène on agite pendant une nuit une suspension composée de 1,15g (4,77 mmol) de 2,3,3a,4-tetrahydro-1*H*-pyrrolo[2,1-*c*][1,2,4]benzothiadiazin-7-ol 5,5-dioxide, 1,00 g (6,21 mmol) d'acide [4-(cyanomethyl)phenyl]boronique, 1,3 g (7,15 mmol), d'acétate de cuivre (II), 1,16 ml (14,31 mmol) de pyridine et d'environ 500 mg de tamis moléculaire 4 Å. Le milieu réactionnel est dilué en ajoutant 100 ml supplémentaires de chlorure de méthylène et on filtre la suspension. Le filtrat est concentré puis directement déposé sur une colonne de silice que l'on élue avec un système 99/1 chlorure de méthylène/méthanol. Les fractions contenant le produit attendu sont rassemblés, évaporés et le résidu est repris dans un peu d'éther éthylique. Après filtration du solide, le produit du titre est obtenu sous forme de poudre beige.
*Point de fusion : 156-158 °C*

### Stade B : 7-[4-(1H-Tétrazol-5-ylméthyl)phénoxy]-2,3,3a,4-tétrahydro-1H-pyrrolo [2,1-c][1,2,4]benzothiadiazine 5,5-dioxide

On agite 24 h à 110°C une suspension de 300 mg (0,844 mmol) du produit obtenu au stade A, 164 mg (2,53 mmol) d'azidure de sodium et 113 mg (2,11 mmol) de NH₄Cl dans 3 ml de DMF. On laisse revenir à température ambiante et verse le milieu réactionnel dans 20 ml de HCl 1N. On extrait (AcOEt), sèche (MgSO₄), et évapore à sec. Le résidu est trituré dans Et₂O et le précipité est filtré pour conduire au produit du titre.
*Point de fusion : 209-212 °C*

**Micro-analyse élémentaire :**

| | *C* | *H* | *N* | *S* |
|---|---|---|---|---|
| *% théorique* | *54,26* | *4,55* | *21,09* | *8,05* |
| *% expérimental* | *54,18* | *4,44* | *20,67* | *8,05* |

### EXEMPLE 28 : 3-{4-[(5,5-Dioxido-2,3,3a,4-tétrahydro-1H-pyrrolo[2,1-c][1,2,4] benzothiadiazin-7-yl)oxy]benzyl}-1,2,4-oxadiazole-5(4H)-thione

### Stade A : 2-{4-[(5,5-Dioxido-2,3-dihydro-1H-pyrrolo[2,1-c][1,2,4]benzothiadiazin-7-yl)oxy]phényl}-N'-hydroxyéthanimidamide

A une solution de 1,25 g (18,0 mmol) du chlorhydrate d'hydroxylamine dans 4 ml de DMSO on ajoute 2,51 ml (18,0 mmol) de triéthylamine et agite la suspension 10 min. Le précipité est filtré et le filtrat est concentré. A ce filtrat on ajoute ensuite 992 mg (3,00 mmol) du produit du stade A de l'exemple 27 et on agite la solution à 75°C pendant 1h 30. On laisse revenir à température ambiante et précipite le milieu réactionnel dans l'eau. Le précipité est filtré pour conduire au produit du titre.

### Stade B : 3-{4-[(5,5-Dioxido-2,3-dihydro-1H-pyrrolo[2,1-c][1,2,4]benzothiadiazin-7-yl)oxy]benzyl}-1,2,4-oxadiazole-5(4H)-thione

A une suspension du produit obtenu au stade A (330 mg, 0,85 mmol) dans 8 ml de CH₃CN, on ajoute 348 mg (1,95 mmol) de 1,1'-thiocarbonyldiimidazole puis 530 µl (3,516 mmol) de DBU. La solution réactionnelle est agitée une nuit à température ambiante. On ajoute 20 ml de HCl 1N, extrait (CH₂Cl₂), lave (NaCl saturé), sèche (MgSO₄), évapore à sec. Le produit du titre est obtenu sous forme d'une cire jaune que l'on engage brut dans le stade suivant.

### Stade C : 3-{4-[(5,5-Dioxido-2,3,3a,4-tétrahydro-1H-pyrrolo[2,1-c][1,2,4] benzothiadiazin-7-yl)oxy]benzyl}-1,2,4-oxadiazole-5(4H)-thione

On agite à température ambiante le produit obtenu au stade B (290 mg, 0,68 mmol) dans l'éthanol (12 ml) en présence de NaBH₄ (77 mg, 2,03 mmol) pendant 1 h. On ajoute 10 ml d'HCl 1N et extrait (CH₂Cl₂). Le produit du titre est purifié par chromatographie sur colonne de silice (CH₂Cl₂/MeOH 99/1).
*Point de fusion : 124-126 °C*

**Micro-analyse élémentaire :**

| | *C* | *H* | *N* | *S* |
|---|---|---|---|---|
| *% théorique* | *53,01* | *4, 21* | *13,01* | *14,90* |
| *% expérimental* | *53,05* | *4,37* | *12,32* | *15,20* |

### EXEMPLE 29 : N-(1-{4-[(5,5-Dioxido-2,3,3a,4-tétrahydro-1H-pyrrolo[2,1-c][1,2,4] benzothiadiazin-7-yl)oxy]phényl}éthyl)méthanesulfonamide

### Stade A : 1-{4-[(5,5-Dioxido-2,3,3a,4-tétrahydro-1H-pyrrolo[2,1-c][1,2,4] benzothiadiazin-7-yl)oxy]phényl}éthanone

Dans 150 ml de chlorure de méthylène on agite pendant une nuit une suspension composée de 3,0 g (12,48 mmol) de 2,3,3a,4-tétrahydro-1*H*-pyrrolo[2,1-*c*][1,2,4]benzothiadiazin-7-ol 5,5-dioxide, 3,18 g (18,73 mmol) d'acide (4-acétylphényl)boronique, 3,42 g (18,82 mmol) d'acétate de cuivre (II), 3,03 ml (37,15 mmol) de pyridine et environ 500 mg de tamis moléculaire 4 Å. Le milieu réactionnel est dilué en ajoutant 100 ml supplémentaires de chlorure de méthylène et on filtre la suspension. Le filtrat est concentré puis directement déposé sur une colonne de silice que l'on élue avec un système 99/1 CH₂Cl₂/acétone. Les fractions contenant le produit attendu sont rassemblées, évaporées et le résidu est repris dans de l'éther éthylique. Après filtration du solide on récupère le produit du titre sous forme de poudre blanche.
*Point de fusion : 152-154°C*

### Stade B : (1-{4-[(5,5-Dioxido-2,3,3a,4-tétrahydro-1H-pyrrolo[2,1-c][1,2,4] benzothiadiazin-7-yl)oxy]phényl}éthyl)amine

A une solution de 1,0 g (2,80 mmol) du produit obtenu au stade A dans 5 ml de méthanol ammoniacal 7N on ajoute goutte à goutte 1,65 ml (5,63 mmol) de titanium(IV)isopropoxide. On agite une nuit à température ambiante, ajoute 424 mg (11,20 mmol) de NaBH₄ et poursuit 2 h l'agitation. On précipite le milieu réactionnel par addition d'eau (2-3 ml), filtre un précipité blanc. Le filtrat est mis de côté. Le précipité est suspendu dans AcOEt, on agite 30 min et filtre. Le filtrat est joint au premier et on poursuit l'extraction avec AcOEt. Les phases organiques sont rassemblées, lavées (NaCl saturé), séchées (MgSO₄), évaporées sous vide pour conduire au produit du titre.

### Stade C : N-(1-{4-[(5,5-Dioxido-2,3,3a,4-tétrahydro-1H-pyrrolo[2,1-c][1,2,4] benzothiadiazin-7-yl)oxy]phényl}éthyl)méthanesulfonamide

On procède comme dans l'exemple 2 avec pour produit de départ le produit du stade B précédent.
*Point de fusion : 122-127 °C*

**Micro-analyse élémentaire :**

| | *C* | *H* | *N* | *S* |
|---|---|---|---|---|
| *% théorique* | *52,16* | *5,30* | *9, 60* | *14, 66* |
| *% expérimental* | *51,93* | *5,81* | *9,32* | *14,59* |

### EXEMPLE 30 : N-(1-{3-[(5,5-Dioxido-2,3,3a,4-tétrahydro-1H-pyrrolo[2,1-c][1,2,4] benzothiadiazin-7-yl)oxy]phényl}éthyl)méthanesulfonamide

On procède comme dans les stades A, B et C de l'exemple 29 en remplaçant au stade A l'acide (4-acétylphényl)boronique par l'acide (3-acétylphényl)boronique.
*Point de fusion : 83-84* °C

**Micro-analyse élémentaire :**

| | *C* | *H* | *N* | *S* |
|---|---|---|---|---|
| *% théorique* | *52,16* | *5,30* | *9,60* | *14,66* |
| *% expérimental* | *52,03* | *5.28* | *9,20* | *14,81* |

### EXEMPLE 31 : N-{4-[(5,5-Dioxido-2,3,3a,4-tétrahydro-1H-pyrrolo[2,1-c][1,2,4] benzothiadiazin-7-yl)oxy]benzyl}-N-méthylméthanesulfonamide

Dans 50 ml de CH₂Cl₂ on agite pendant une nuit une suspension composée de 5,74 mg (3,37 mmol) de 2,3,3a,4-tétrahydro-1*H*-pyrrolo[2,1-*c*][1,2,4]benzothiadiazin-7-ol 5,5-dioxide, 926 mg (4,04 mmol) d'acide (4-{[méthyl(méthylsulfonyl)amino]méthyl} phényl)boronique, 920 mg (5,06 mmol), d'acétate de cuivre (II), 817 µl (10,10 mmol) de pyridine et d'environ 4 g de tamis moléculaire 4 Å. Le milieu réactionnel est filtré, rincé avec CH₂Cl₂/MeOH (1/1). Le filtrat est concentré puis directement déposé sur une colonne de silice que l'on élue avec un système 95/5 CH₂Cl₂/MeOH. Les fractions contenant le produit attendu sont rassemblées, évaporées et le résidu est repris dans de l'éther éthylique. Après filtration du solide on récupère le produit du titre sous forme de poudre blanche.
*Point de fusion: 142-144°C*

**Micro-analyse élémentaire :**

| | *C* | *H* | *N* | *S* |
|---|---|---|---|---|
| *% théorique* | *52,16* | *5,30* | *9, 60* | *14, 66* |
| *% expérimental* | *51,99* | *5,55* | *9,43* | *14,86* |

### EXEMPLE 32 : {3-[(5,5-Dioxido-2,3,3a,4-tétrahydro-1H-pyrrolo[2,1-c][1,2,4] benzothiadiazin-7-yl)oxy]benzyl}diméthylamine

### Stade A : 3-[(5,5-Dioxido-2,3-dihydro-1H-pyrrolo[2,1-c][1,2,4]benzothiadiazin-7-yl) oxy]benzoate de méthyle

On procède comme au stade B de l'exemple 1 en prenant comme produit de départ le composé obtenu au stade A de l'exemple 1 et l'acide [3-(méthoxycarbonyl) phényl]boronique.
*Point de fusion : 211-214°C*

**Micro-analyse élémentaire :**

| | *C* | *H* | *N* | *S* |
|---|---|---|---|---|
| *% théorique* | *58, 06* | *4,33* | *7,52* | *8, 61* |
| *% expérimental* | *57,70* | *4,54* | *7,29* | *8,37* |

### Stade B : Acide 3-[3-(aminosulfonyl)-4-(2-oxopyrrolidin-1-yl)phénoxy]benzoïque

On chauffe à 95°C une suspension du produit obtenu au stade A (1,1g, 2,55 mmol) dans 18 ml de NaOH 1N jusqu'à obtenir une solution. On laisse revenir à température ambiante, acidifie avec HCl 1N et extrait (CH₂Cl₂). Les phases organiques sont rassemblées, lavées (NaCl saturé), séchées (MgSO₄) et évaporées. Le résidu est trituré dans Et₂O et le produit du titre précipite et est récupéré par filtration.

### Stade C : Acide 3-[(2,3-dihydro-5,5-dioxido-1H-pyrrolo[2,1-c][1,2,4]benzothiadiazin-7-yl)oxy] benzoïque

On chauffe 1 h au reflux une suspension du produit obtenu au stade B (850 mg, 2,26 mmol) dans 25 ml de THF en présence de 675 µl (4,52 mmol) de DBU. On laisse revenir à température ambiante, acidifie avec HCl 1N et filtre le précipité blanc qui correspond au produit du titre.

**Micro-analyse élémentaire :**

| | *C* | *H* | *N* | *S* |
|---|---|---|---|---|
| *% théorique* | *56,98* | *3,94* | *7,82* | *8,95* |
| *% expérimental* | *57,15* | *4,13* | *7, 68* | *9,16* |

### Stade D : 3-[(5,5-Dioxido-2,3-dihydro-1H-pyrrolo[2,1-c][1,2,4]benzothiadiazin-7-yl)oxy]-N,N-diméthylbenzamide :

A une suspension du produit obtenu au stade C (1,40 g, 3,91 mmol) dans 20 ml de CH₂Cl₂ on ajoute 2 gouttes de DMF puis goutte à goutte 684 µl (7,81 mmol) de chlorure d'oxalyle dilué dans 2 ml de CH₂Cl₂. On agite 2h 30 à température ambiante, évapore à sec, reprend dans 15 ml de CH₂Cl₂, ajoute 1,1 ml (7,81 mmol) de Et₃N puis 2,94 ml (5,87 mmol) d'une solution 2 M de diméthylamine dans le THF. On agite 1h à température ambiante. Le milieu réactionnel est acidifié avec HCl 0,5N et on extrait (CH₂Cl₂). Les phases organiques sont rassemblées, lavées (NaCl saturé), séchées (MgSO₄) et évaporées. Le résidu est trituré dans Et₂O et le produit du titre précipite et est récupéré par filtration

**Micro-analyse élémentaire :**

| | *C* | *H* | *N* | *S* |
|---|---|---|---|---|
| *% théorique* | *59,21* | *4,97* | *10,90* | *8,32* |
| *% expérimental* | *59,23* | *5,09* | *10,47* | *7,97* |

### Stade E : {3-[(5,5-Dioxido-2,3,3a,4-tétrahydro-1H-pyrrolo[2,1-c][1,2,4] benzothiadiazin-7-yl)oxy]benzyl}diméthylamine

A une suspension de 577 mg (1,49 mmol) du produit obtenu au stade D dans 20 ml de THF on ajoute goutte à goutte 3,72 ml (3,72 mmol) d'une solution 1M de LiAlH₄ dans le THF. On observe un passage en solution de la réaction et on poursuit l'agitation 3 h à température ambiante. L'excès d'hydrure est hydrolysé par addition goutte à goutte d'eau jusqu'à l'arrêt de dégagement gazeux. On agite 10 min la suspension, ajoute 20 ml d'eau et extrait avec AcOEt. Les phases organiques sont rassemblées, lavées (NaCl saturé), séchées (MgSO₄), évaporées et le résidu est chromatographié sur colonne de silice (CH₂Cl₂/MeOH 95/5) pour conduire au produit du titre.
*Point de fusion : 122°C*

**Micro-analvse élémentaire :**

| | *C* | *H* | *N* | *S* |
|---|---|---|---|---|
| *% théorique* | *61,10* | *6,21* | *11,25* | *8,59* |
| *% expérimental* | *61,32* | *6,19* | *11,06* | *8,52* |

### ETUDE PHARMACOLOGIQUE DES PRODUITS DE L'INVENTION

### Etude des courants excitateurs induit par l'AMPA dans les oocytes de Xenopus

### a - Méthode :

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.
L'enregistrement électrophysiologique est réalisé dans une chambre en plexiglass^{®} à 20-24°C en milieu OR2 (J. Exp. Zool., 1973, 184, 321-334) par la méthode du "voltage-clamp" à 2 électrodes, une 3ème électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b - Résultats :

Les composés de l'invention potentialisent très fortement les effets excitateurs de l'AMPA et leur activité est très nettement supérieure à celle des composés de référence. Le composé de l'exemple 1 possède notamment une EC2X égale à 3,5 µM et une EC5X égale à 9,2 µM, le composé de l'exemple 2 une EC2X égale à 0,35 µM et une EC5X égale à 2,6 µM, et le composé de l'exemple 21 une EC2X égale à 0,1 µM et une EC5X égale à 0,56 µM.

**COMPOSITION PHARMACEUTIQUE**

| | |
|---|---|
| Formule de préparation pour 1000 comprimés dosés à 100 mg de *N*-{3-[(5,5-Dioxido-2,3,3a,4-tétrahydro-1*H*-pyrrolo[2,1-*c*][1,2,4]benzothiadiazin-7-yl)oxy]benzyl}méthanesulfonamide (Exemple 2) | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 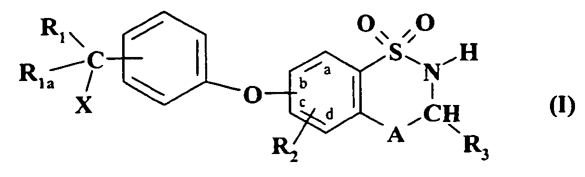 dans laquelle :
**R**_{**1**} représente un atome d'hydrogène, d'halogène ou un groupement alkyle (C₁-C₆) linéaire ou ramifié,
**R**_{**1a**} représente un atome d'hydrogène ou un groupement alkyle (C₁-C₆) linéaire ou ramifié,
**R**_{**2**} représente un atome d'hydrogène, d'halogène ou un groupement hydroxy,
**A** représente un groupement CR₄R₅ ou un groupement NR₄,
**R**_{**3**} représente un atome d'hydrogène, un groupement alkyle (C₁-C₆) linéaire ou ramifié ou un groupement cycloalkyle (C₃-C₇),
**R**_{**4**} représente un atome d'hydrogène ou un groupement alkyle (C₁-C₆) linéaire ou ramifié,
ou bien
**A** représente un atome d'azote et forme avec le groupement -CHR₃- adjacent le cycle 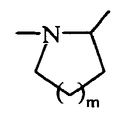 dans lequel m représente 1, 2 ou 3,
**R**_{**5**} représente un atome d'hydrogène ou d'halogène,
**X** représente un groupement NR₆R₇, S(O)ₙR₈, OR'₈ ou un groupement hétérocyclique dans lesquels :
**R**_{**6**} représente un atome d'hydrogène, un groupement alkyle (C₁-C₆) linéaire ou ramifié, S(O)ₚR₉, COR₉ ou P(O)OR₉OR₁₀,
**R**_{**7**} représente un atome d'hydrogène, ou un groupement alkyle (C₁-C₆) linéaire ou ramifié,
ou bien **R**_{**6**} et **R**_{**7**} forment ensemble avec l'atome d'azote qui les porte un groupement hétérocyclique,
**R**_{**8**}**, R**_{**9**} et **R**_{**10**}**,** identiques ou différents, représentent un atome d'hydrogène ; un groupement alkyle (C₁-C₆) linéaire ou ramifié éventuellement substitué par un ou plusieurs atomes d'halogène ; un groupement arylalkyle (C₁-C₆) linéaire ou ramifié ; ou un groupement aryle,
**R'**_{**8**} représente un groupement alkyle (C₁-C₆) linéaire ou ramifié ou acyle (C₁-C₆) linéaire ou ramifié,
**n et p,** identiques ou différents, représentent 1 ou 2,
leurs énantiomères et diastéréoisomères ainsi que leurs sels d'addition à un acide ou à une base pharmaceutiquement acceptable.

2. Composés de formule (I) selon la revendication 1 tels que R₂ représente un atome d'hydrogène.

3. Composés de formule (I) selon l'une quelconque des revendications 1 ou 2 tels que le groupement 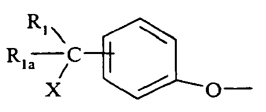 est en position b du phényle qui le porte.

4. Composés de formule (I) selon l'une quelconque des revendications 1, 2 ou 3 tels que X est un groupement NR₆R₇, S(O)ₙR₈ ou un groupement hétérocyclique.

5. Composés de formule (I) selon l'une quelconque des revendications 1, 2, 3 ou 4 tels que X est un groupement NR₆R₇.

6. Composés de formule (I) selon l'une quelconque des revendications 1 à 5 tels que 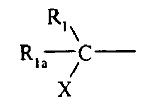 est en position méta du noyau phénoxy qui le porte.

7. Composés de formule (I) selon l'une quelconque des revendications 1 à 5 tels que 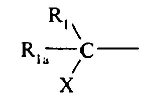 est en position para du noyau phénoxy qui le porte.

8. Composés de formule (I) selon l'une quelconque des revendications 1 à 7 tels que A représente un atome d'azote et forme avec le groupement ―CHR₃― adjacent le cycle 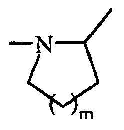 dans lequel m représente 1, 2 ou 3, et préférentiellement 1.

9. Composé de formule (I) selon la revendication 1 qui est le {3-[(5,5-dioxido-2,3,3a,4-tétrahydro-1*H*-pyrrolo[2,1-*c*][1,2,4]benzothiadiazin-7-yl)oxy]phényl}méthanamine ainsi que ses sels d'addition.

10. Composé de formule (I) selon la revendication 1 qui est le *N*-{3-[(5,5-dioxido-2,3,3a,4-tétrahydro-1*H*-pyrrolo[2,1-*c*][1,2,4]benzothiadiazin-7-yl)oxy]benzyl}méthanesulfonamide ainsi que ses sels d'addition.

11. Composé de formule (I) selon la revendication 1qui est le *N*-{4-[(5,5-dioxido-2,3,3a,4-tétrahydro-1*H*-pyrrolo[2,1-*c*][1,2,4]benzothiadiazin-7-yl)oxy]benzyl}méthanesulfonamide ainsi que ses sels d'addition.

12. Procédé de préparation des composés de formule (I) selon la revendication 1 dans laquelle A représente un groupement NR₄ ou A représente un atome d'azote et forme avec le groupement CHR₃ adjacent le cycle 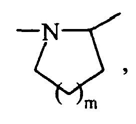 dans lequel m représente 1, 2 ou 3, **caractérisé en ce que** l'on utilise comme produit de départ un composé de formule **(II)** : 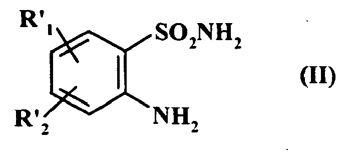 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui est :
**(a) soit mis en réaction** avec le chlorure d'acide de formule **(III)** en présence d'une base, en milieu tétrahydrofurane ou acétonitrile :
**Cl - CH**_{**2**} **- (CH**_{**2**}**)**_{**m**} **- CH**_{**2**} **- COCl** **(III)**
dans laquelle m a la même signification que dans la formule (I),
pour conduire au composé de formule **(IV) :** 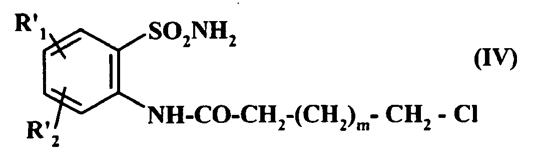 dans laquelle R'₁ et R'₂ sont tels que définis précédemment,
qui est alors cyclisé en milieu basique, pour conduire au composé de formule **(V) :** 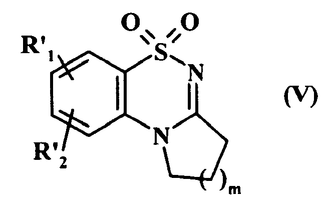 dans laquelle R'₁, R'₂ et m sont tels que définis précédemment,
qui subit éventuellement une réduction, en milieu alcoolique ou diméthylformamide, en présence de borohydrure de sodium, pour conduire au composé de formule **(VI)** : 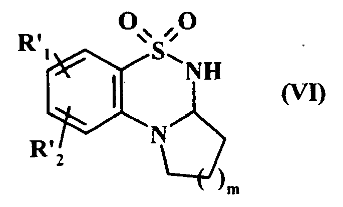 dans laquelle R'₁, R'₂ et m sont tels que définis précédemment,
composé de formule **(V)** ou **(VI)** qui subit l'action du tribromure de bore, pour conduire au composé de formule **(VII) :** 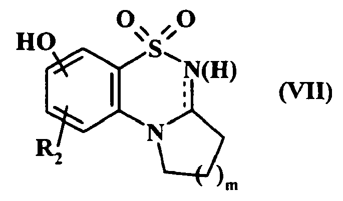 dans laquelle R₂ est tel que défini dans la formule (I) et m est tel que défini précédemment,
**(b) soit cyclisé :**
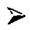 en présence d'une amidine de formule **(VIII)** : 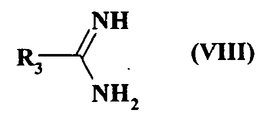 dans laquelle R₃ est tel que défini dans la formule (I),
pour conduire au composé de formule **(IX)** : 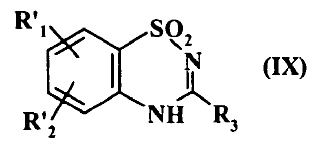 dans laquelle R'₁, R'₂, et R₃ ont la même signification que précédemment,
qui est :
◆ ou bien réduit par un hydrure métallique,
pour conduire au composé de formule **(X)** : 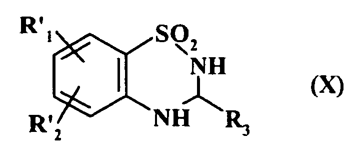 dans laquelle R'₁, R'₂, et R₃ ont la même signification que précédemment,
◆ ou bien alkylé par action d'une base forte en présence d'un agent d'alkylation R'₄X dans lequel R'₄ représente un groupement alkyle (C₁-C₆) linéaire ou ramifié et X un atome d'halogène, puis réduit,
pour conduire au composé de formule **(XI)** : 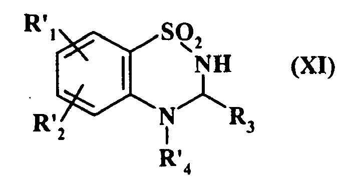 dans laquelle R'₁, R'₂, R₃ et R'₄ ont la même signification que précédemment,
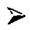 en présence d'un aldéhyde de formule **(XII)** : 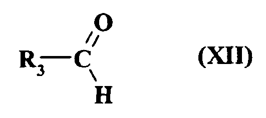 dans laquelle R₃ est tel que défini dans la formule (I),
pour conduire au composé de formule (X) décrit précédemment,
composé de formule (X) ou (XI),
dont on transforme le groupement R'₁ et le groupement R'₂ lorsque celui-ci représente un groupement alkoxy (C₁-C₆) linéaire ou ramifié, en groupements hydroxy,
pour conduire au composé de formule **(XIII) :** 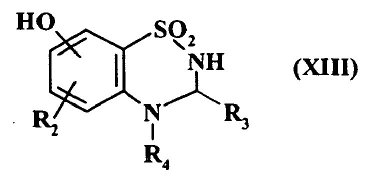 dans laquelle R₂, R₃ et R₄ ont la même signification que dans la formule (I),
composé de formule (VII) ou (XIII) que l'on fait réagir avec un dérivé d'acide boronique de formule **(XIV) :** 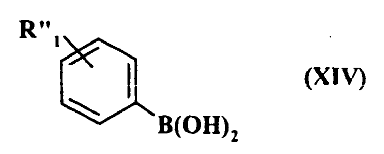 dans laquelle R"₁ représente un groupement cyano ou un groupement R₁R₁ₐXC- tel que défini dans la formule (I),
pour conduire, (après transformation éventuelle du groupement R"₁ quand celui-ci représente un groupement cyano en groupement NR₆R₇ tel que défini dans la formule (I)), au composé de formule **(I/a**_{**1**}**)** ou **(I/a**_{**2**}**),** cas particuliers des composés de formule (I) : 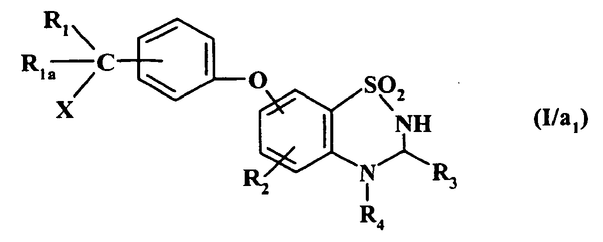 dans laquelle R₁, R₁ₐ, R₂, R₃, R₄ et X ont la même signification que dans la formule (I), 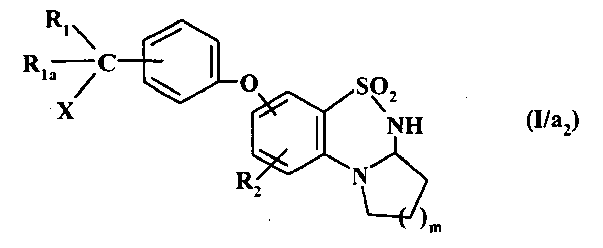 dans laquelle R₁, R₁ₐ, R₂, m et X ont la même signification que dans la formule (I),
composés de formule (I/a₁) ou (I/a₂) :
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

13. Procédé de préparation des composés de formule (I) selon la revendication 1 dans laquelle A représente un groupement CR₄R₅ **caractérisé en ce que** l'on utilise comme produit de départ, un composé de formule **(XV)** : 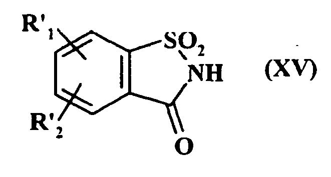 dans laquelle :
R'₁ représente un groupement alkoxy (C₁-C₆) linéaire ou ramifié,
R'₂ représente un atome d'hydrogène, d'halogène ou un groupement alkoxy (C₁-C₆) linéaire ou ramifié,
qui subit l'action de la chloroacétone en présence de diméthylformamide, pour conduire au composé de formule **(XVI)** : 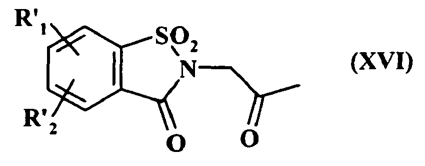 dans laquelle R'₁ et R'₂ ont la même signification que précédemment,
qui subit un réarrangement en milieu basique, pour conduire au composé de formule (**XVII**) : 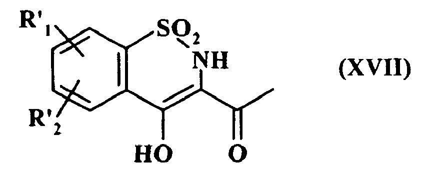 dans laquelle R'₁ et R'₂ ont la même signification que précédemment,
qui est déacétylé par chauffage au reflux en milieu benzénique en présence d'un excès d'éthylèneglycol et d'une quantité catalytique d'acide p-toluène sulfonique, pour conduire au composé de formule (**XVIII**) : 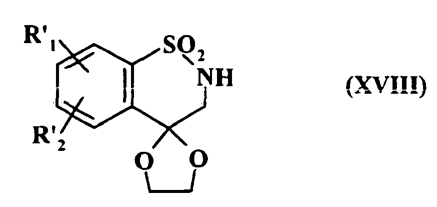 dans laquelle R'₁ et R'₂ ont la même signification que précédemment,
qui subit une hydrolyse en milieu acide, pour conduire au composé de formule **(XIXa)** : 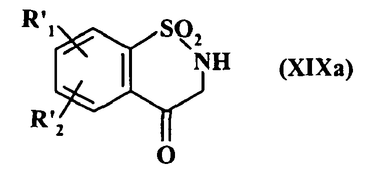 dans laquelle R'₁ et R'₂ ont la même signification que précédemment,
dont, éventuellement, selon la nature du groupement R₃ que l'on souhaite obtenir, on protège l'atome d'azote par un groupement protecteur, puis, après traitement par une base forte, que l'on traite par un composé de formule R'₃-P,
dans laquelle R'₃ représente un groupement alkyle (C₁-C₆) linéaire ou ramifié ou un groupement cycloalkyle (C₃-C₇) et P représente un groupe partant,
pour conduire après déprotection de l'atome d'azote, au composé de formule **(XIX'a)** : 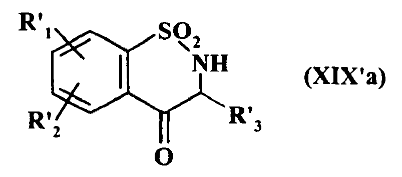 dans laquelle R'₁, R'₂ et R'₃ ont la même signification que précédemment,
composé de formule (XIXa) et (XIX'a) représentés par la formule **(XIX)** : 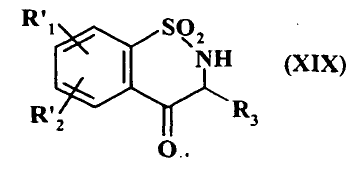 dans laquelle R'₁ et R'₂ ont la même signification que précédemment et R₃ est tel que défini dans la formule (I), qui :
- **soit** subit une réduction catalytique, pour conduire au composé de formule **(XX) :** 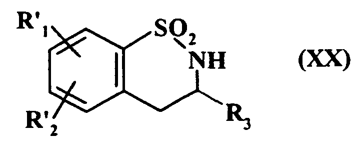 dans laquelle R'₁ et R'₂ ont la même signification que précédemment,
- **soit** est transformé par l'action d'un hydrure en alcool dont on transforme le groupement hydroxy en atome d'halogène par l'action d'un réactif approprié,
pour conduire au composé de formule **(XXI)** : 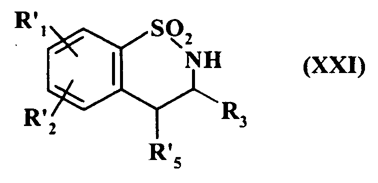 dans laquelle R'₁, R'₂ et R₃ ont la même signification que précédemment, et R'₅ représente un atome d'halogène,
- **soit** subit l'action d'un organomagnésien R'₄MgBr dans lequel R'₄ représente un groupement alkyle (C₁-C₆) linéaire ou ramifié,
pour conduire au composé de formule **(XIXb) :** 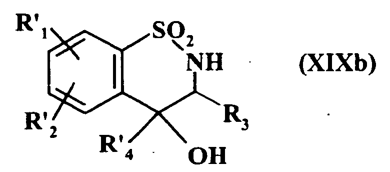 dans laquelle R'₁, R'₂, R₃ et R'₄ ont la même signification que précédemment,
composé de formule **(XIXb)** :
- **ou bien** qui subit une réduction catalytique, pour conduire au composé de formule **(XXII) :** 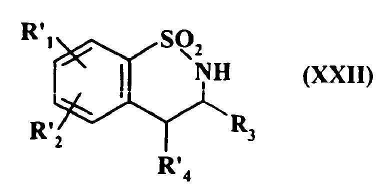 dans laquelle R'₁, R'₂, R₃ et R'₄ ont la même signification que précédemment,
- **ou bien** dont on transforme le groupement hydroxy en atome d'halogène par l'action d'un réactif approprié,
pour conduire au composé de formule **(XXIII) :** 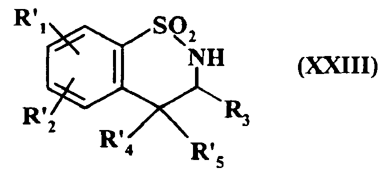 dans laquelle R'₁, R'₂, R₃ et R'₄ ont la même signification que précédemment et R'₅ représente un atome d'halogène,
composé de formule **(XX)** à **(XXIII)** dont on transforme le groupement R'₁ et le groupement R'₂ lorsque celui-ci représente un groupement alkoxy (C₁-C₆) linéaire ou ramifié, en groupements hydroxy,
pour conduire au composé de formule **(XXIV) :** 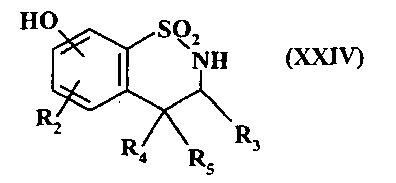 dans laquelle R₂, R₃, R₄ et R₅ ont la même signification que dans la formule (I),
composé de formule **(XXIV),**
que l'on fait réagir avec un dérivé d'acide boronique de formule **(XIV) :** 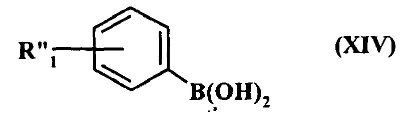 dans laquelle R"₁ représente un groupement cyano ou un groupement R₁R₁ₐXC- tel que défini dans la formule (I),
pour conduire, (après transformation éventuelle du groupement R"₁ quand celui-ci représente un groupement cyano en groupement NR₆R₇ tel que défini dans la formule (I)), au composé de formule **(I/b)**, cas particuliers des composés de formule (I) : 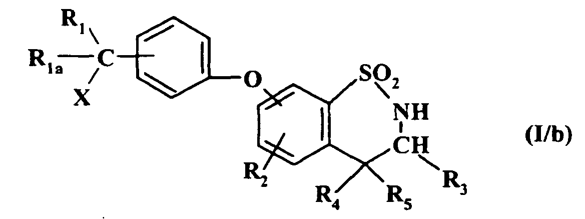 dans laquelle R₁, R₁ₐ, R₂, R₃, R₄, R₅ et X ont la même signification que dans la formule (I),
composé de formule (I/b), que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

14. Compositions pharmaceutiques contenant comme principe actif un composé selon l'une quelconque des revendications 1 à 11 en combinaison avec un ou plusieurs excipients ou véhicules inertes, non toxiques, pharmaceutiquement acceptables.

15. Compositions pharmaceutiques selon la revendication 14 contenant comme principe actif un composé selon l'une quelconque des revendications 1 à 11 utiles en tant que médicaments, comme modulateurs AMPA.

## Claims

1. Compounds of formula (I) : 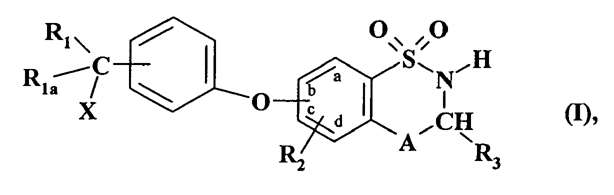 wherein:
**R**_{**1**} represents a hydrogen atom, a halogen atom or a linear or branched (C₁-C₆)alkyl group,
**R**_{**1a**} represents a hydrogen atom or a linear or branched (C₁-C₆)alkyl group,
**R**_{**2**} represents a hydrogen atom, a halogen atom or a hydroxy group,
**A** represents a CR₄R₅ group or an NR₄ group,
**R**_{**3**} represents a hydrogen atom, a linear or branched (C₁-C₆)alkyl group or a (C₃-C₇)cycloalkyl group,
**R**_{**4**} represents a hydrogen atom or a linear or branched (C₁-C₆)alkyl group,
or
**A** represents a nitrogen atom and, together with the adjacent -CHR₃- group, forms the ring 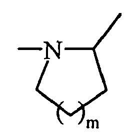 wherein m represents 1, 2 or 3,
**R**_{**5**} represents a hydrogen or halogen atom,
**X** represents an NR₆R₇, S(O)ₙR₈ or OR'₈ group or a heterocyclic group, wherein :
**R**_{**6**} represents a hydrogen atom, a linear or branched (C₁-C₆)alkyl group, S(O)ₚR₉, COR₉ or P(O)OR₉OR₁₀,
**R**_{**7**} represents a hydrogen atom or a linear or branched (C₁-C₆)alkyl group,
or **R**_{**6**} and **R**_{**7**}**,** together with the nitrogen atom carrying them, form a heterocyclic group,
**R**_{**8**}**, R**_{**9**} and **R**_{**10**}**,** which may be the same or different, represent a hydrogen atom; a linear or branched (C₁-C₆)alkyl group optionally substituted by one or more halogen atoms; an aryl-(C₁-C₆)alkyl group in which the alkyl moiety is linear or branched; or an aryl group,
**R'**_{**8**} represents a linear or branched (C₁-C₆)alkyl group or a linear or branched (C₁-C₆)acyl group,
**n** and **p,** which may be the same or different, represent 1 or 2,
their enantiomers and diastereoisomers, and also addition salts thereof with a pharmaceutically acceptable acid or base.

2. Compounds of formula (I) according to claim 1, wherein R₂ represents a hydrogen atom.

3. Compounds of formula (I) according to either claim 1 or claim 2, wherein the grouping 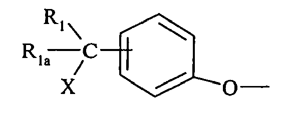 is in position b of the phenyl carrying it.

4. Compounds of formula (I) according to any one of claims 1, 2 and 3, wherein X is an NR₆R₇ or S(O)ₙR₈ group or a heterocyclic group.

5. Compounds of formula (I) according to any one of claims 1, 2, 3 and 4, wherein X is an NR₆R₇ group.

6. Compounds of formula (I) according to any one of claims 1 to 5, wherein 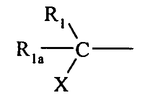 is in the meta-position of the phenoxy ring structure carrying it.

7. Compounds of formula (I) according to any one of claims 1 to 5, wherein 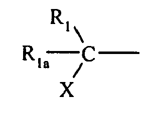 is in the para-position of the phenoxy ring structure carrying it.

8. Compounds of formula (I) according to any one of claims 1 to 7, wherein A represents a nitrogen atom and, together with the adjacent ―CHR₃― group, forms the ring 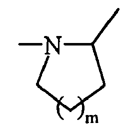 wherein m represents 1, 2 or 3, preferably 1.

9. Compound of formula (I) according to claim 1, which is {3-[(5,5-dioxido-2,3,3a,4-tetrahydro-1*H*-pyrrolo[2,1-*c*][1,2,4]benzothiadiazin-7-yl)oxy]phenyl}methanamine, and also addition salts thereof.

10. Compound of formula (I) according to claim 1, which is *N*-{3-[(5,5-dioxido-2,3,3a,4-tetrahydro-1*H*-pyrrolo[2,1-*c*][1,2,4]benzothiadiazin-7-yl)oxy]benzyl}methanesulphonamide, and also addition salts thereof.

11. Compound of formula (I) according to claim 1, which is *N*-{4-[(5,5-dioxido-2,3,3a,4-tetrahydro-1*H*-pyrrolo[2,1-*c*][1,2,4]benzothiadiazin-7-yl)oxy]benzyl}methanesulphonamide, and also addition salts thereof.

12. Process for the preparation of compounds of formula (I) according to claim 1 wherein A represents an NR₄ group or A represents a nitrogen atom and, together with the adjacent CHR₃ group, forms the ring 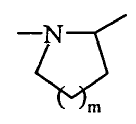 wherein m represents 1, 2 or 3, **characterised in that** there is used as starting material a compound of formula **(II) :** 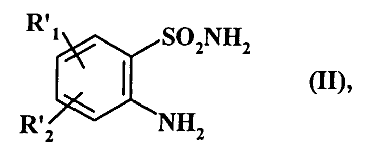 wherein :
R'₁ represents a linear or branched (C₁-C₆)alkoxy group,
R'₂ represents a hydrogen atom, a halogen atom or a linear or branched (C₁-C₆)alkoxy group,
which is :
**(a) either reacted** with the acid chloride of formula **(III)** in the presence of a base, in a tetrahydrofuran or acetonitrile medium :
**Cl-CH**_{**2**} **- (CH**_{**2**}**)**_{**m**} **- CH**_{**2**} **- COCl** **(III),**
wherein m is as defined for formula (I),
to yield the compound of formula **(IV) :** 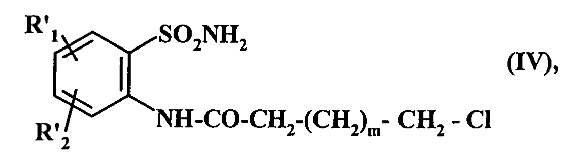 wherein R'₁ and R'₂ are as defined hereinbefore,
which is then cyclised in a basic medium to yield the compound of formula **(V)** : 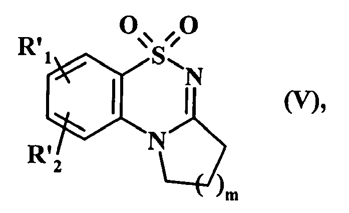 wherein R'₁, R'₂ and m are as defined hereinbefore,
which is optionally subjected to reduction, in an alcoholic or dimethylformamide medium, in the presence of sodium borohydride, to yield the compound of formula **(VI) :** 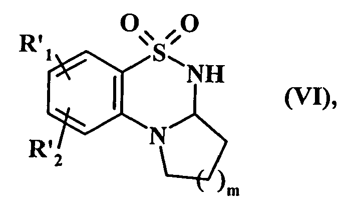 wherein R'₁, R'₂ and m are as defined hereinbefore,
which compound of formula **(V)** or **(VI)** is subjected to the action of boron tribromide to yield the compound of formula **(VII) :** 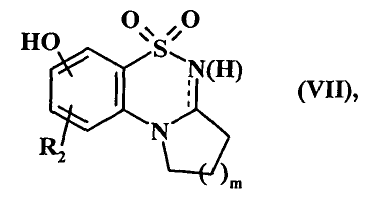 wherein R₂ is as defined for formula (I) and m is as defined hereinbefore,
**(b) or cyclised :**
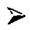 in the presence of an amidine of formula **(VIII) :** 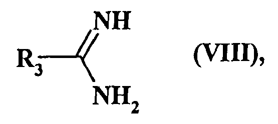
wherein R₃ is as defined for formula (I),
to yield the compound of formula **(IX) :** 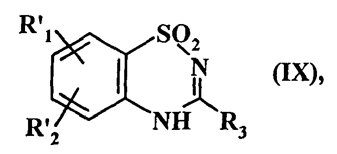 wherein R'₁, R'₂ and R₃ are as defined hereinbefore,
which is :
◆ either reduced, using a metallic hydride,
to yield the compound of formula **(X)** : 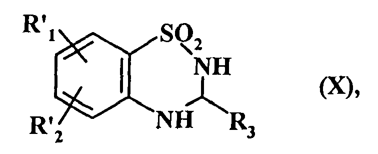 wherein R'₁, R'₂ and R₃ are as defined hereinbefore,
◆ or alkylated by the action of a strong base in the presence of an alkylating agent R'₄X, wherein R'₄ represents a linear or branched (C₁-C₆)alkyl group and X represents a halogen atom, and then reduced
to yield the compound of formula **(XI) :** 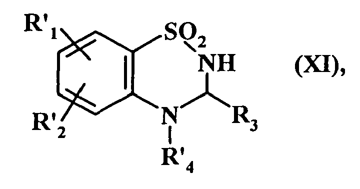 wherein R'₁, R'₂, R₃ and R'₄ are as defined hereinbefore,
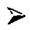 in the presence of an aldehyde of formula **(XII) :** 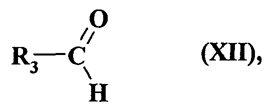 wherein R₃ is as defined for formula (I),
to yield the compound of formula (X) described hereinbefore,
in which compound of formula (X) or (XI)
the group R'₁ and, when the group R'₂ represents a linear or branched (C₁-C₆)alkoxy group, the group R'₂ are converted into hydroxy groups
to yield the compound of formula **(XIII) :** 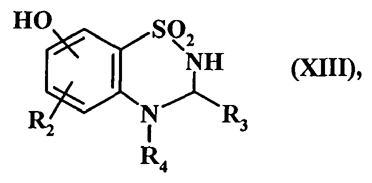 wherein R₂, R₃ and R₄ are as defined for formula (I),
which compound of formula (VII) or (XIII) is reacted with a boronic acid compound of formula **(XIV) :** 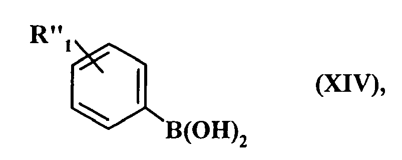 wherein R"₁ represents a cyano group or an R₁R₁ₐXC- group as defined for formula (I),
to yield (after optional conversion of the group R"₁, when the latter represents a cyano group, into an NR₆R₇ group as defined for formula (I)) the compound of formula **(I/a**_{**1**}**)** or **(I/a**_{**2**}**),** particular cases of the compounds of formula (I) : 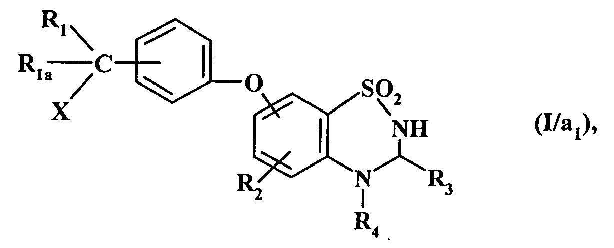 wherein R₁, R₁ₐ, R₂, R₃, R₄ and X are as defined for formula (I), 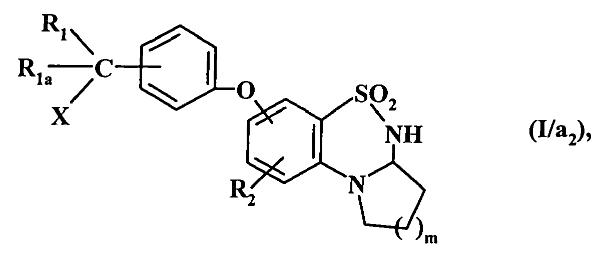 wherein R₁, R₁ₐ, R₂, m and X are as defined for formula (I),
which compounds of formulae (I/a₁) and (I/a₂):
are purified, if necessary, according to a conventional purification technique, are separated, if desired, into their isomers according to a conventional separation technique and are converted, if desired, into their addition salts with a pharmaceutically acceptable acid or base.

13. Process for the preparation of compounds of formula (I) according to claim 1 wherein A represents a CR₄R₅ group, **characterised in that** there is used as starting material a compound of formula **(XV)** : 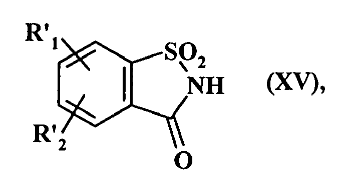 wherein :
R'₁ represents a linear or branched (C₁-C₆)alkoxy group,
R'₂ represents a hydrogen atom, a halogen atom or a linear or branched (C₁-C₆)alkoxy group,
which is subjected to the action of chloroacetone in the presence of dimethylformamide to yield the compound of formula **(XVI) :** 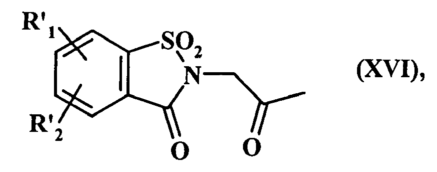
wherein R'₁ and R'₂ are as defined hereinbefore,
which is subjected to a rearrangement in a basic medium to yield the compound of formula **(XVII) :** 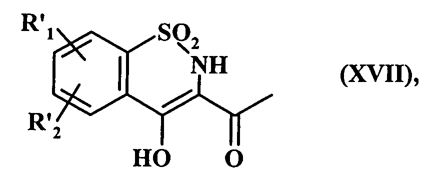 wherein R'₁ and R'₂ are as defined hereinbefore,
which is deacetylated by heating at reflux in a benzene medium in the presence of an excess of ethylene glycol and a catalytic amount of p-toluenesulphonic acid to yield the compound of formula **(XVIII) :** 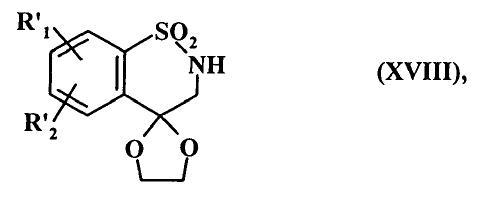 wherein R'₁ and R'₂ are as defined hereinbefore,
which is subjected to hydrolysis in an acid medium to yield the compound of formula **(XIXa) :** 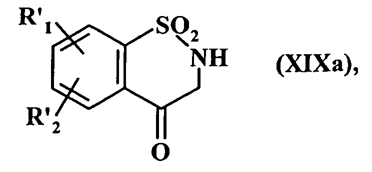 wherein R'₁ and R'₂ are as defined hereinbefore,
the nitrogen atom of which is optionally, depending on the nature of the group R₃ that is desired, protected by a protecting group and which is then, after treatment with a strong base, treated with a compound of formula R'₃-P,
wherein R'₃ represents a linear or branched (C₁-C₆)alkyl group or a (C₃-C₇)cycloalkyl group and P represents a leaving group,
to yield, after deprotection of the nitrogen atom, the compound of formula **(XIX'a) :** 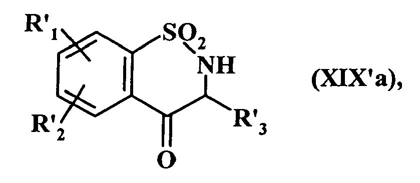 wherein R'₁, R'₂ and R'₃ are as defined hereinbefore,
which compound of formula (XIXa) or (XIX'a), represented by formula **(XIX) :** 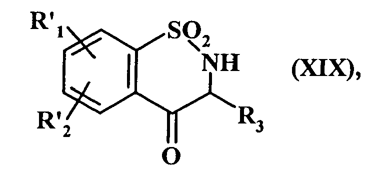 wherein R'₁ and R'₂ are as defined hereinbefore and R₃ is as defined for formula (I), is :
- either subjected to catalytic reduction to yield the compound of formula **(XX)** : 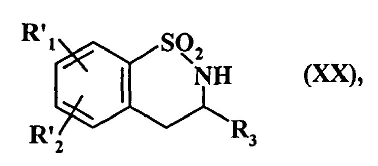 wherein R'₁ and R'₂ are as defined hereinbefore,
- **or** converted by the action of a hydride into an alcohol, the hydroxy group of which is converted into a halogen atom by the action of an appropriate reagent
to yield the compound of formula **(XXI) :** 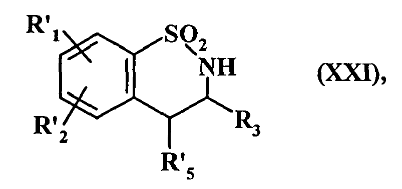 wherein R'₁, R'₂ and R₃ are as defined hereinbefore and R'₅ represents a halogen atom,
- **or** subjected to the action of an organomagnesium compound R'₄MgBr, wherein R'₄ represents a linear or branched (C₁-C₆)alkyl group,
to yield the compound of formula **(XIXb)** : 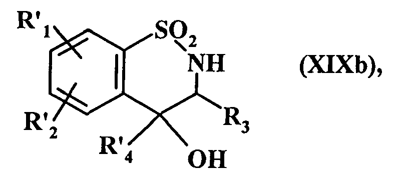 wherein R'₁, R'₂, R₃ and R'₄ are as defined hereinbefore,
which compound of formula **(XIXb)**
- **either** is subjected to catalytic reduction to yield the compound of formula **(XXII) :** 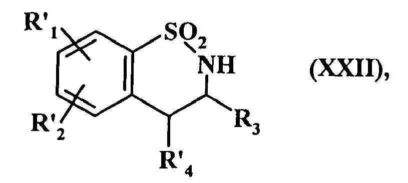 wherein R'₁, R'₂, R₃ and R'₄ are as defined hereinbefore,
- **or** the hydroxy group thereof is converted into a halogen atom by the action of an appropriate reagent
to yield the compound of formula **(XXIII) :** 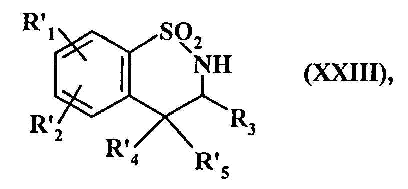
wherein R'₁, R'₂, R₃ and R'₄ are as defined hereinbefore and R'₅ represents a halogen atom,
in which compounds of formulae **(XX)** to **(XXIII)** the group R'₁ and, when the group R'₂ represents a linear or branched (C₁-C₆)alkoxy group, the group R'₂ are converted into hydroxy groups
to yield the compound of formula **(XXIV) :** 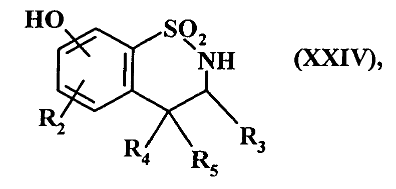 wherein R₂, R₃, R₄ and R₅ are as defined for formula (I),
which compound of formula **(XXIV)**
is reacted with a boronic acid compound of formula **(XIV) :** 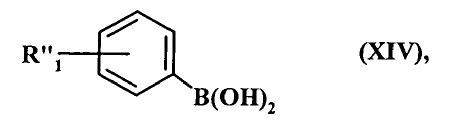 wherein R"₁ represents a cyano group or an R₁R₁ₐXC― group as defined for formula (I),
to yield (after optional conversion of the group R"₁, when the latter represents a cyano group, into an NR₆R₇ group as defined for formula (I)) the compound of formula **(I/b),** a particular case of the compounds of formula (I) : 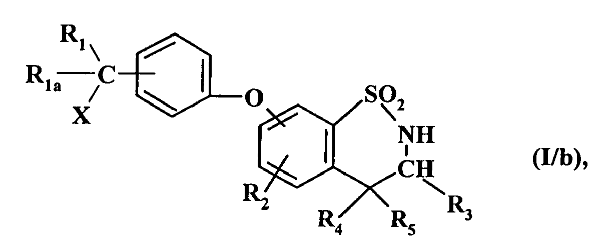 wherein R₁, R₁ₐ, R₂, R₃, R₄, R₅ and X are as defined for formula (I),
which compound of formula (I/b) is purified, if necessary, according to a conventional purification technique, is separated, if desired, into its isomers according to a conventional separation technique and is converted, if desired, into its addition salts with a pharmaceutically acceptable acid or base.

14. Pharmaceutical compositions comprising as active ingredient a compound according to any one of claims 1 to 11 in combination with one or more inert, non-toxic, pharmaceutically acceptable excipients or carriers.

15. Pharmaceutical compositions according to claim 14 comprising as active ingredient a compound according to any of claims 1 to 11, for use as medicaments as AMPA modulators.

## Patentansprüche

1. Verbindungen der Formel (I): 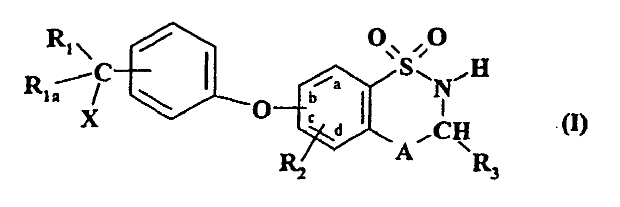 in der:
**R**_{**1**} ein Wasserstoffatom, Halogenatom oder eine geradkettige oder verzweigte (C₁₋C₆)-Alkylgruppe bedeutet,
**R**_{**1a**} ein Wasserstoffatom oder eine geradkettige oder verzweigte (C₁-C₆)-Alkylgruppe bedeutet,
**R**_{**2**} ein Wasserstoffatom, Halogenatom oder eine Hydroxygruppe bedeutet,
**A** eine Gruppe CR₄R₅ oder eine Gruppe NR₄ bedeutet,
**R**_{**3**} ein Wasserstoffatom, eine geradkettige oder verzweigte (C₁-C₆)-Alkylgruppe oder eine (C₃-C₇)-Cycloalkylgruppe bedeutet,
**R**_{**4**} ein Wasserstoffatom oder eine geradkettige oder verzweigte (C₁-C₆)-Alkylgruppe bedeutet,
oder
**A** ein Stickstoffatom bedeutet und zusammen mit der benachbarten Gruppe -CHR₃- den Ring bildet 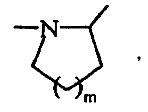 in dem m den Wert 1, 2 oder 3 besitzt,
**R**_{**5**} ein Wasserstoffatom oder ein Halogenatom bedeutet,
**X** eine Gruppe NR₆R₇, S(O)ₙR₈, OR'₈ oder eine heterocyclische Gruppe bedeutet, worin:
**R**_{**6**} ein Wasserstoffatom, eine geradkettige oder verzweigte (C₁-C₆)-Alkylgruppe, S(O)ₚR₉, COR₉ oder P(O)OR₉OR₁₀ darstellt,
**R**_{**7**} ein Wasserstoffatom oder eine geradkettige oder verzweigte (C₁-C₆)-Alkylgruppe darstellt, oder
**R**_{**6**} und **R**_{**7**} gemeinsam mit dem sie tragenden Stickstoffatom eine heterocyclische Gruppe bilden,
**R**_{**8**}**,** **R**_{**9**} und **R**_{**10**}**,** die gleichartig oder verschieden sind, ein Wasserstoffatom; eine geradkettige oder verzweigte (C₁-C₆)-Alkylgruppe, die gegebenenfalls durch ein oder mehrere Halogenatome substituiert ist; eine geradkettige oder verzweigte Aryl-(C₁-C₆)-alkylgruppe; oder eine Arylgruppe darstellen,
**R'**_{**8**} eine geradkettige oder verzweigte (C₁-C₆)-Alkylgruppe oder eine geradkettige oder verzweigte (C₁-C₆)-Acylgruppe darstellt, und
**n und p,** die gleichartig oder verschieden sind, 1 oder 2 darstellen,
deren Enantiomere und Diastereoisomere sowie deren Additionssalze mit einer pharmazeutisch annehmbaren Säure oder Base.

2. Verbindungen der Formel (I) nach Anspruch 1, worin R₂ ein Wasserstoffatom bedeutet.

3. Verbindungen der Formel (I) nach einem der Ansprüche 1 oder 2, worin die Gruppe 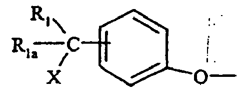 in der Stellung b des sie tragenden Phenylrests steht.

4. Verbindungen der Formel (I) nach einem der Ansprüche 1, 2 oder 3, worin X eine Gruppe NR₆R₇, S(O)ₙR₈ oder eine heterocyclische Gruppe bedeutet.

5. Verbindungen der Formel (I) nach einem der Ansprüche 1, 2, 3 oder 4, worin X eine Gruppe NR₆R₇ bedeutet.

6. Verbindungen der Formel (I) nach einem der Ansprüche 1 bis 5, worin 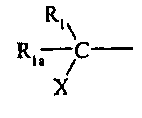 in der meta-Stellung des sie tragenden Phenoxykerns steht.

7. Verbindungen der Formel (I) nach einem der Ansprüche 1 bis 5, worin 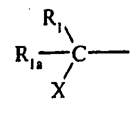 in der para-Stellung des sie tragenden Phenoxykerns steht.

8. Verbindungen der Formel (I) nach einem der Ansprüche 1 bis 7, worin A ein Stickstoffatom bedeutet und zusammen mit der benachbarten Gruppe -CHR₃- den Ring 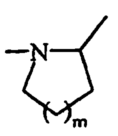 bildet, in dem m 1, 2 oder 3 und vorzugsweise 1 bedeutet.

9. Verbindung der Formel (I) nach Anspruch 1, nämlich {3-[(5,5-Dioxido-2,3,3a,4-tetrahydro-1*H*-pyrrolo[2,1-*c*][1,2,4]benzothiadiazin-7-yl)-oxy]-phenyl}-methanamin sowie dessen Additionssalze.

10. Verbindung der Formel (I) nach Anspruch 1, nämlich *N*-{3-[(5,5-Dioxido-2,3,3a,4-tetrahydro-1*H*-pyrrolo[2,1-*c*][1,2,4]benzothiadiazin-7-yl)-oxy]-benzyl}-methansulfonamid sowie dessen Additionssalze.

11. Verbindung der Formel (I) nach Anspruch 1, nämlich *N*-{4-[(5,5-Dioxido-2,3,3a,4-tetrahydro-1*H*-pyrrolo[2,1-*c*][1,2,4]benzothiadiazin-7-yl)-oxy]-benzyl}-methansulfonamid sowie dessen Additionssalze.

12. Verfahren zur Herstellung der Verbindungen der Formel (I) nach Anspruch 1, worin A eine Gruppe NR₄ bedeutet oder A ein Stickstoffatom bedeutet und zusammen mit der benachbarten Gruppe -CHR₃- den Ring 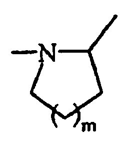 bildet, in dem m 1, 2 oder 3 bedeutet, **dadurch gekennzeichnet, daß** man als Ausgangsprodukt eine Verbindung der Formel **(II)** verwendet: 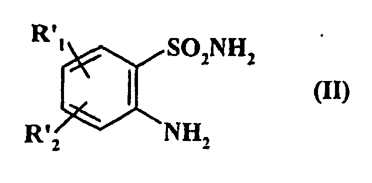 in der:
R'₁ eine geradkettige oder verzweigte (C₁-C₆)-Alkoxygruppe und
R'₂ ein Wasserstoffatom, Halogenatom oder eine geradkettige oder verzweigte (C₁₋C₆)-Alkoxygruppe bedeuten,
welche:
(a) **entweder umgesetzt wird** mit dem Säurechlorid der Formel **(III)**:
**Cl - CH**_{**2**} **- (CH**_{**2**}**)**_{**m**} **- CH**_{**2**} **- COCI** **(III)**
in der m die bezüglich der Formel (I) angegebene Bedeutung besitzt, in Gegenwart einer Base in Tetrahydrofuran- oder Acetonitril-Medium zur Bildung der Verbindung der Formel (IV): 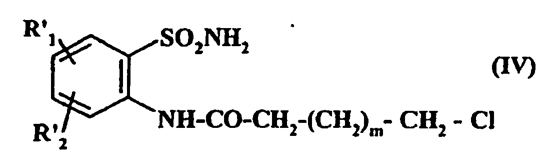 in der R'₁ und R'₂ die oben angegebenen Bedeutungen besitzen,
welche anschließend in basischem Medium cyclisiert wird zur Bildung der Verbindung der Formel (V): 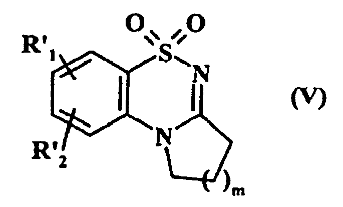 in der R'₁, R'₂ und m die oben angegebenen Bedeutungen besitzen,
welche man gegebenenfalls einer Reduktion in alkoholischem Medium oder in Dimethylformamid in Gegenwart von Natriumborhydrid unterzieht zur Bildung der Verbindung der Formel **(VI):** 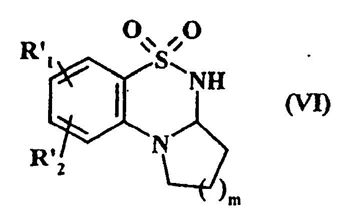 in der R'₁, R'₂ und m die oben angegebenen Bedeutungen besitzen,
welche Verbindung der Formel **(V)** oder **(VI)** man der Einwirkung von Bortribromid unterwirft
zur Bildung der Verbindung der Formel **(VII)**: 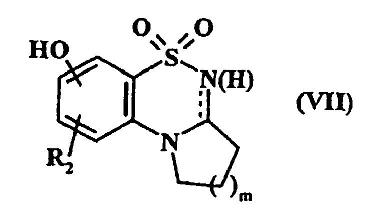 in der R₂ die bezüglich der Formel (I) angegebenen Bedeutungen besitzt und m die oben angegebenen Bedeutungen aufweist,
**(b) oder cyclisiert wird:**
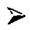 in Gegenwart eines Amidins der Formel **(VIII):** 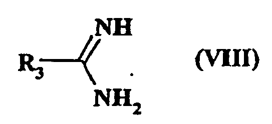 in der R₃ die bezüglich der Formel (I) angegebenen Bedeutungen besitzt, zur Bildung der Verbindung der Formel **(IX):** 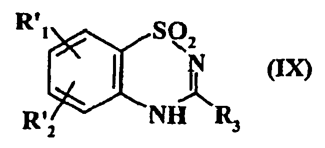 in der R'₁, R'₂ und R₃ die oben angegebenen Bedeutungen besitzen, welche:
◆ entweder mit einem Metallhydrid reduziert wird, zur Bildung der Verbindung der Formel (X): 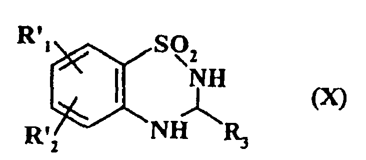 in der R'₁, R'₂ und R₃ die oben angegebenen Bedeutungen besitzen,
◆ oder alkyliert wird durch Einwirkung einer starken Base in Gegenwart eines Alkylierungsmittels R'₄X, worin R'₄ eine geradkettige oder verzweigte (C₁-C₆)-Alkylgruppe und X ein Halogenatom bedeuten, und dann reduziert wird, zur Bildung der Verbindung der Formel **(XI):** 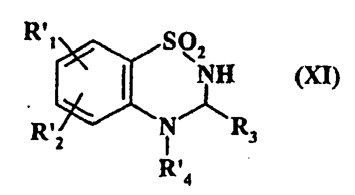 in der R'₁, R'₂, R₃ und R'₄ die oben angegebenen Bedeutungen besitzen,
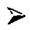 in Gegenwart eines Aldehyds der Formel **(XII):** 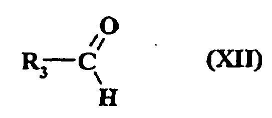 in der R₃ die bezüglich der Formel (I) angegebenen Bedeutungen besitzt,
zur Bildung der oben beschriebenen Verbindung der Formel (X),
bei welcher Verbindung der Formel (X) oder (XI)
man die Gruppe R'₁ und die Gruppe R'₂, wenn diese eine geradkettige oder verzweigte (C₁-C₆)-Alkoxygruppe darstellt, in Hydroxygruppen umwandelt,
zur Bildung der Verbindung der Formel **(XIII):** 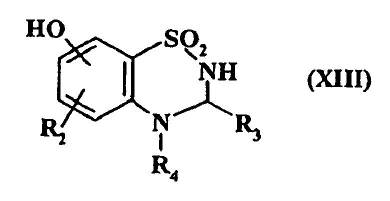 in der R₂, R₃ und R₄ die bezüglich der Formel (I) angegebenen Bedeutungen besitzen,
welche Verbindung der Formel (VII) oder (XIII) man mit einem Boronsäurederivat der Formel **(XIV)** umsetzt: 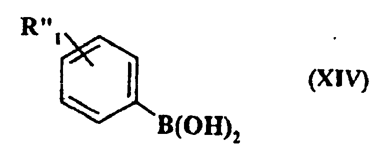 in der R"₁ eine Cyanogruppe oder eine Gruppe R₁R₁ₐXC-, wie sie bezüglich der Formel (I) definiert worden ist, bedeutet,
zur Bildung (nach der eventuellen Umwandlung der Gruppe R"₁, wenn diese eine Cyanogruppe darstellt, in die Gruppe NR₆R₇, wie sie oben bezüglich der Formel (I) definiert worden ist) der Verbindung der Formel **(I/a**_{**1**}**)** oder **(I/a**_{**2**}**),** Sonderfällen der Verbindungen der Formel (I): 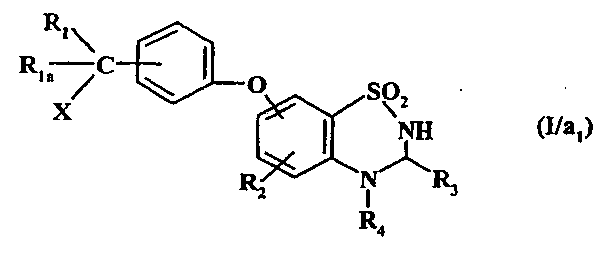 in der R₁, R₁ₐ, R₂, R₃, R₄ und X die bezüglich der Formel (I) angegebenen Bedeutungen besitzen, 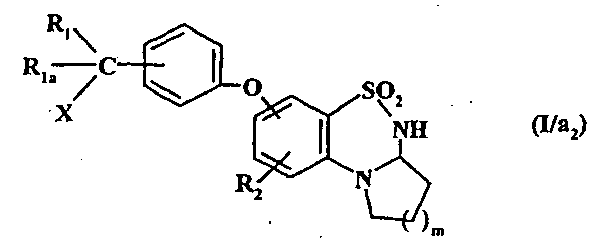 in der R₁, R₁ₐ, R₂, m und X die bezüglich der Formel (I) angegebenen Bedeutungen besitzen,
welche Verbindungen der Formeln (I/a₁) oder (I/a₂) man gegebenenfalls mit Hilfe einer klassischen Reinigungsmethode reinigt, man gegebenenfalls mit Hilfe einer klassischen Trennmethode in ihre Isomeren auftrennt und welche man gewünschtenfalls in ihre Additionssalze mit einer pharmazeutisch annehmbaren Säure oder Base überführt.

13. Verfahren zur Herstellung der Verbindungen der Formel (I) nach Anspruch 1, worin A eine Gruppe CR₄R₅ bedeutet, **dadurch gekennzeichnet, daß** man als Ausgangsprodukt eine Verbindung der Formel (XV) verwendet: 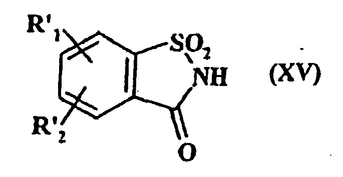 in der:
R'₁ eine geradkettige oder verzweigte (C₁-C₆)-Alkoxygruppe und
R'₂ ein Wasserstoffatom, Halogenatom oder eine geradkettige oder verzweigte (C₁₋C₆)-Alkoxygruppe bedeuten,
welche man der Einwirkung von Chloraceton in Gegenwart von Dimethylformamid unterwirft zur Bildung der Verbindung der Formel **(XVI):** 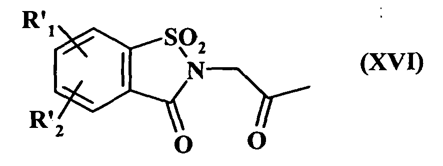 in der R'₁ und R'₂ die oben angegebenen Bedeutungen besitzen,
welche man einer Umlagerung in basischem Medium unterzieht zur Bildung der Verbindung der Formel **(XVII):** 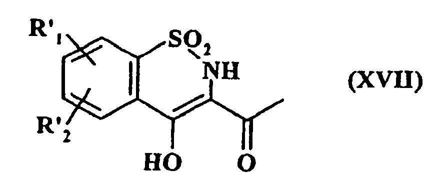 in der R'₁ und R'₂ die oben angegebenen Bedeutungen besitzen,
welche durch Erhitzen am Rückfluß in Benzolmedium in Gegenwart eines Überschusses von Ethylenglykol und einer katalytischen Menge *p*-Toluolsulfonsäure deacetyliert wird zur Bildung der Verbindung der Formel **(XVIII):** 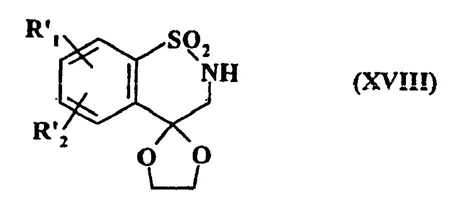 in der R'₁ und R'₂ die oben angegebenen Bedeutungen besitzen,
welche man in saurem Medium hydrolysiert zur Bildung der Verbindung der Formel **(XIXa):** 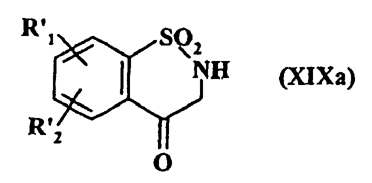 in der R'₁ und R'₂ die oben angegebenen Bedeutungen besitzen,
bei der man gegebenenfalls in Abhängigkeit von der Art der Gruppe R₃, die man herzustellen wünscht, das Stickstoffatom mit einer Schutzgruppe schützt, und
dann nach der Behandlung mit einer starken Base mit einer Verbindung der Formel R'₃-P umsetzt,
in der R'₃ eine geradkettige oder verzweigte (C₁-C₆)-Alkylgruppe oder eine (C₃-C₇)-Cycloalkylgruppe und P eine austretende Gruppe bedeuten,
so daß man nach der Abspaltung der Schutzgruppe des Stickstoffatoms die Verbindung der Formel **(XIX'a)** erhält: 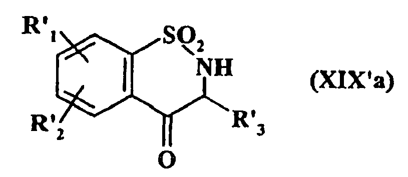 in der R'₁, R'₂ und R'₃ die oben angegebenen Bedeutungen besitzen,
welche Verbindungen der Formel (XIXa) und (XIX'a), die durch die Formel **(XIX)** wiedergegeben werden: 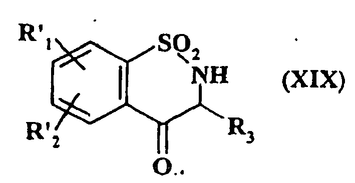 in der R'₁ und R'₂ die oben angegebenen Bedeutungen besitzen und R₃ die bezüglich der Formel (I) angegebenen Bedeutungen besitzt, man:
- **entweder** einer katalytischen Reduktion unterzieht zur Bildung der Verbindung der Formel **(XX)**: 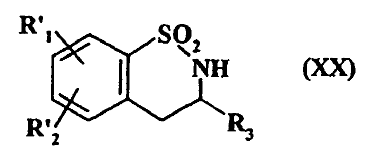 in der R'₁ und R'₂ die oben angegebenen Bedeutungen besitzen,
- **oder** durch Einwirkung eines Hydrids in einen Alkohol überführt, dessen Hydroxygruppe man durch Einwirkung eines geeigneten Reagens in ein Halogenatom umwandelt,
zur Bildung der Verbindung der Formel **(XXI):** 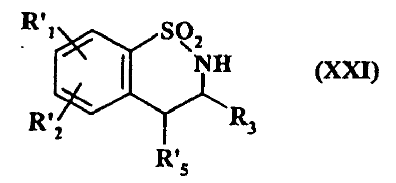 in der R'₁, R'₂ und R₃ die oben angegebenen Bedeutungen besitzen und R'₅ ein Halogenatom darstellt,
- **oder** der Einwirkung einer magnesiumorganischen Verbindung R'₄MgBr, in der R'₄ eine geradkettige oder verzweigte (C₁-C₆)-Alkylgruppe darsellt, unterwirft, zur Bildung der Verbindung der Formel **(XIXb):** 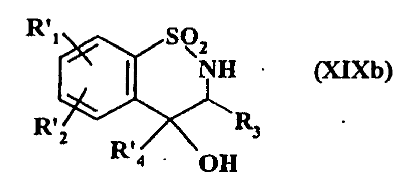 in der R'₁, R'₂, R₃ und R'₄ die oben angegebenen Bedeutungen besitzen, welche Verbindung der Formel **(XIXb)** man:
- **entweder** einer katalytischen Reduktion unterwirft zur Bildung der Verbindung der Formel **(XXII):** 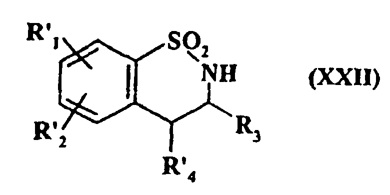 in der R'₁, R'₂, R₃ und R'₄ die oben angegebenen Bedeutungen besitzen,
- **oder** deren Hydroxygruppe man durch Einwirkung eines geeigneten Reagens in ein Halogenatom umwandelt, zur Bildung der Verbindung der Formel **(XXIII):** 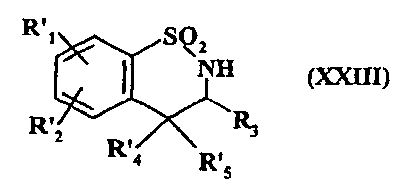 in der R'₁, R'₂, R₃ und R'₄ die oben angegebenen Bedeutungen besitzen und R'₅ ein Halogenatom darstellt,
von welchen Verbindungen der Formeln (**XX**) bis **(XXIII)** man die Gruppe R'₁ und
die Gruppe R'₂, wenn diese eine geradkettige oder verzweigte (C₁-C₆)-Alkoxygruppe darstellt, in Hydroxygruppen umwandelt, zur Bildung der Verbindung der Formel **(XXIV):** 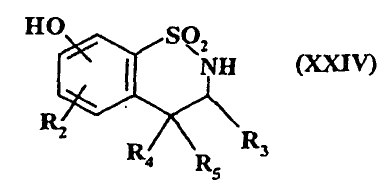 in der R₂, R₃, R₄ und R₅ die bezüglich der Formel (I) angegebenen Bedeutungen besitzen,
welche Verbindung der Formel **(XXIV)**
man mit einem Boronsäurederivat der Formel **(XIV):** 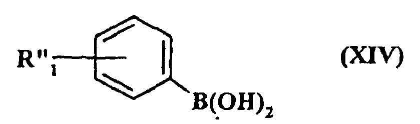 in der R"₁ eine Cyanogruppe oder eine Gruppe R₁R₁ₐXC-, wie sie bezüglich der Formel (I) definiert worden ist, bedeutet, umsetzt
zur Bildung (nach der eventuellen Umwandlung der Gruppe R"₁, wenn diese eine Cyanogruppe bedeutet, in die Gruppe NR₆R₇, wie sie bezüglich der Formel (I) definiert worden ist) der Verbindung der Formel **(I/b),** Sonderfällen der Verbindungen der Formel (I): 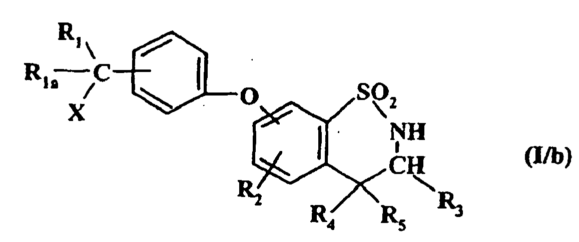 in der R₁, R₁ₐ, R₂, R₃, R₄, R₅ und X die bezüglich der Formel (I) angegebenen Bedeutungen besitzen,
welche Verbindung der Formel (I/b) man gegebenenfalls mit Hilfe einer klassischen Reinigungsmethode reinigt, man gegebenenfalls mit Hilfe einer klassischen Trennmethode in ihre Isomeren auftrennt und gewünschtenfalls in ihre Additionssalze mit einer pharmazeutisch annehmbaren Säure oder Base umwandelt.

14. Pharmazeutische Zubereitungen enthaltend als Wirkstoff eine Verbindung nach einem der Ansprüche 1 bis 11 in Kombination mit einem oder mehreren inerten, nichttoxischen, pharmazeutisch annehmbaren Trägermaterialien oder Hilfsstoffen.

15. Pharmazeutische Zubereitungen nach Anspruch 14, enthaltend als Wirkstoff eine Verbindung nach einem der Ansprüche 1 bis 11 nützlich als Arzneimittel, als AMPA-Modulatoren.
